# EUROPEAN PATENT APPLICATION

(11) **EP 4 209 230 A1**
(43) Date of publication of application: **12.07.2023**
(21) Application number: 21863537.3
(22) Date of filing: 23.08.2021
(51) Int. Cl.: A61K 47/69, A61K 38/28, A61K 45/00, A61K 47/54, A61P 3/10, B82Y 5/00

(54) **MESOPOROUS SILICA NANOPARTICLE CONTROLLED RELEASE SYSTEM, PREPARATION METHOD THEREFOR AND APPLICATION THEREOF**

(30) Priority: 03.09.2020 CN 202010914907
(71) Applicant: East China University of Science and Technology, Shanghai 200237 (CN)
(72) Inventor: ZHU, Weiping, Shanghai 200237 (CN); FU, Yun, Shanghai 200237 (CN); XU, Yufang, Shanghai 200237 (CN); QIAN, Xuhong, Shanghai 200237 (CN)
(74) Representative: Taylor, Gail
(86) International application number: PCT/CN2021/114083
(87) International publication number: WO 2022/048470

(57) **Abstract**

The present invention relates to a mesoporous silica nanoparticle controlled release system, a preparation method thereof and an application thereof, and specifically provides a mesoporous silica nanomaterial that can be used for controlled release. The mesoporous silica nanomaterial that can be used for controlled release comprises a mesoporous silica nanoparticle functionalized by means of a polyhydroxy compound and a nanoparticle plugging agent functionalized by means of a phenylboronic acid compound. When substrate concentration is relatively low, the nanomaterial achieves "zero early release"; when substrate concentration is relatively high, mesoporous pores blocked by the plugging agent are opened and active ingredients are released. The nanomaterial can be loaded into microneedles and microneedle array patches used to treat diabetes or to control blood sugar levels in patients.

## Description

### Technical Field

The present disclosure belongs to the field of controlled release of a drug, specifically relates to a controlled release system of mesoporous silica nanoparticles, a preparation method thereof and its application in a microneedle patch.

### Background

Inorganic porous materials are widely used in catalysts and adsorption carriers due to their large specific surface area and adsorption capacity. According to the pore size, porous materials can be divided into microporous, mesoporous and macroporous materials. The pore size of inorganic microporous materials including wollastonite, activated carbon, zeolite, etc. is generally <2 nm, and the small pore size of microporous materials limits its catalytic and adsorption effects on organic macromolecules. The pore size of macroporous materials including porous ceramics, cement, aerogels, etc. is generally >50nm, and macroporous materials are characterized by a large pore size but a wide distribution range. Those with pore sizes therebetween are called mesoporous materials. Mesoporous materials have the characteristics of extremely high specific surface area, regular and orderly pore channel structure, narrow pore size distribution, continuously adjustable pore size, and no physiological toxicity, and have good application prospects in fields of immobilization and separation of enzymes, proteins and the like, and embedding and controlled release of biochips, drug, etc.

The application of mesoporous silica materials in drug delivery has been extensively studied. Surface modification of mesoporous silica can achieve controlled release of the internal load, which makes it an ideal drug delivery carrier (Chem. Soc. Rev., 2012, 41(7): 2590-2605). The mesoporous silica drug delivery platform is mainly composed of three parts: mesoporous silica, a pore blocking agent and a sensitive response unit. The mesoporous silica with functionalized surface and blocked pore ports has the characteristics of "zero early release", which has attracted extensive attention of researchers, and is known as an effective stimulus-response controlled release system.

Due to an unstable structure of biomacromolecules, their activity is easily affected by environmental factors, such as temperature, pH and the like. Therefore, in the field of biomacromolecule transport, how to maintain the activity of biomacromolecules, improve bioavailability and controllable release is the focus of current research. Some biomacromolecule transport systems constructed by utilizing the protection characteristics of mesopores in mesoporous silica to the structural integrity of biomacromolecules have been partially reported. However, due to a large size of biomacromolecules, mesoporous silica with a correspondingly large size is required to meet the loading requirements, and the resulting particle size of mesoporous silica will increase with the increase of the pore size. However, when this type of mesoporous silica is applied in vivo, it is difficult to pass through the biofilm, and it is easy to be taken up by the reticuloendothelial phagocytic system, and the drug bioavailability is low.

At present, there is no reports on the use of mesoporous silica delivery system with a mesopore diameter of ≥ 5 nm, a mesoporous silica size of less than 350 nm, and relatively independent mesopores, especially the delivery system for biological macromolecules.

Microneedle patch is a means of transdermal drug delivery with great potential. Polysaccharide-based dissolved microneedle patches have the advantages of high drug delivery efficiency, high drug loading rate, convenient manufacturing process, and easy storage. The combination of microneedle patches with an insulin drug delivery system, has broad application prospects.

### Summary

The present disclosure adopts surface-functionalized mesoporous silica nanoparticles with polyhydroxy compounds as a carrier, and surface-functionalized nanoparticles with phenylboronic acid which match the size of the mesopores as a pore blocking agent. Based on the principle of competitive binding between phenylboronic acid ester formed by polyhydroxy compounds and phenylboronic acid and substrate, a controlled release system is designed and synthesized, which can be used for the controlled release of biological macromolecules such as insulin.

Specifically, the present disclosure provides a mesoporous silica nanomaterial for controlled release, wherein the mesoporous silica nanomaterial for controlled release comprises mesoporous silica nanoparticles functionalized by a polyhydroxy compound and nanoparticle pore blocking agents functionalized by phenylboronic acid. The phenylboronic acid group forms a linking group with the hydroxyl group of the polyhydroxy compound, thereby connecting the nanoparticle pore blocking agent functionalized by phenylboronic acid to the mesoporous silica nanoparticles functionalized by a polyhydroxy compound.

In one or more embodiments, the present disclosure can be used in mesoporous silica nanomaterials for controlled release, wherein the nanoparticle pore blocking agent is directly connected to the phenylboronic acid group or connected to the phenylboronic acid group through a linker. The phenylboronic acid group reacts with the hydroxyl group of the polyhydroxy compound to form a linking group, and the polyhydroxy compound is then connected to the mesoporous silica nanoparticles through another linker.

In one or more embodiments, the particle size of the mesoporous silica nanoparticles is 90-350 nm.

In one or more embodiments, the mesopore size of the mesoporous silica nanoparticles is 5-20 nm.

In one or more embodiments, the polyhydroxy compound is selected from the group consisting of monosaccharides and their derivatives, polysaccharides and their derivatives and synthetic block copolymers, preferably from glucose and its derivatives.

In one or more embodiments, the polyhydroxy compound is gluconic acid.

In one or more embodiments, the mesoporous silica nanoparticles functionalized by the polyhydroxy compound have a structure shown in the following formula I: 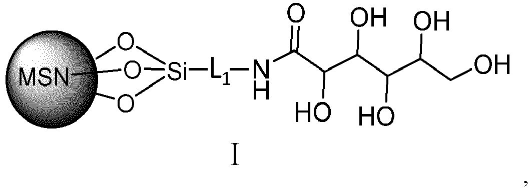 wherein MSN refers to mesoporous silica nanoparticles; L₁ is a linker group; preferably, L₁ is a C₁₋₆ alkylene group, preferably a propylene group.

In one or more embodiments, the size of the nanoparticle pore blocking agent matches the size of mesopores of the mesoporous silica nanoparticles.

In one or more embodiments, the nanoparticle pore blocking agent has a size of 3-25 nm.

In one or more embodiments, the nanoparticle pore blocking agent is selected from a group consisting of metal oxide nanoparticles, metal sulfide nanoparticles, metal nanoparticles, quantum dots, block copolymers, natural polymers and biomacromolecules, preferably metal oxide nanoparticles, and more preferably zinc oxide nanoparticles.

In one or more embodiments, the nanoparticle pore blocking agent functionalized by phenylboronic acid has a structure shown in the following formula III or IV: 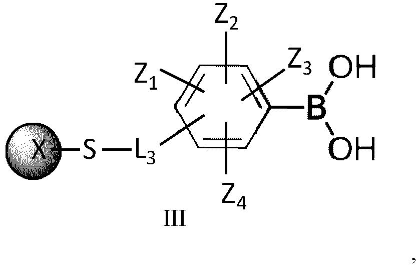 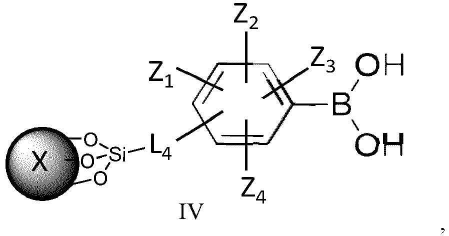 wherein X refers to the nanoparticle pore blocking agent; Z₁, Z₂, Z₃ and Z₄ are each independently selected from a group consisting of hydrogen, electron-withdrawing group substituents and electron-donating group substituents, preferably each independently selected from a group consisting of hydrogen, C₁₋₆ alkyl, halogen, nitro, carboxyl and amino, more preferably each independently selected from a group consisting of hydrogen, C₁₋₆ alkyl and halogen; L₃ and L₄ are each independently absent or is a linker group, preferably, the linker group is selected from a group consisting of: C₁₋₆ alkylene, -(CH₂)ₙ-NR'-CO-aryl-N=N-aryl-NR'-(CH₂)ₒ-, -(CH₂)ₙ-NR'-COO-(CH₂)ₘ-aryl-(CH₂)ₒ-NR'-(CH₂)ₚ-, -(CH₂)ₙ-NR'-CO-, -(CH₂)ₙ-CO-NR'-, -(CH₂)ₙ-NR'-aryl-(CH₂)ₘ-, -(CH₂)ₙ-NR'-(CH₂)ₘ-aryl-(CH₂)ₒ-, -(CH₂)ₙ-NR'-(CH₂)ₘ-aryl-N=N-aryl-(CH₂)ₒ-, and -C₂₋₆ alkenylene-aryl-(CH₂)ₒ-; wherein n, m, o and p are each independently an integer of 0-4; R' is H or C₁₋₄ alkyl; the aryl is selected from a group consisting of phenyl, naphthyl, anthracenyl and phenanthrenyl, and is optionally substituted by 1-3 substituents selected from a group consisting of halogen, C₁₋₄ alkyl and C₁₋₄ alkoxy.

Preferably, the L₃ and L₄ are each independently absent, or each independently selected from a group consisting of: -(CH₂)ₙ-NR'-CO-aryl-N=N-aryl-NR'-(CH₂)ₒ-, -(CH₂)ₙ-NR'-COO-(CH₂)ₘ-aryl-(CH₂)ₒ-NR'-(CH₂)ₚ- and -amido-C₁₋₆ alkylene-; wherein n, m, o and p are each independently an integer of 0-4; R' is H or C₁₋₄ alkyl; the aryl is selected from a group consisting of phenyl, naphthyl, anthracenyl and phenanthrenyl, and is optionally substituted by 1-3 substituents selected from a group consisting of halogen, C₁₋₄ alkyl and C₁₋₄ alkoxy.

In formulas III and IV, the relative positions of Z₁, Z₂, Z₃, Z₄, L₃, L₄ and the phenylboronic acid group on the benzene ring are not limited, that is, L₃ or L₄ can be located in meta, para or ortho position of the phenylboronic acid group.

In one or more embodiments, the nanoparticle pore blocking agent functionalized by phenylboronic acid is: 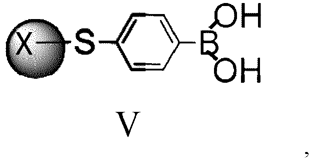 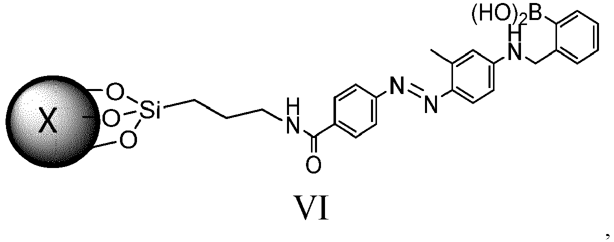 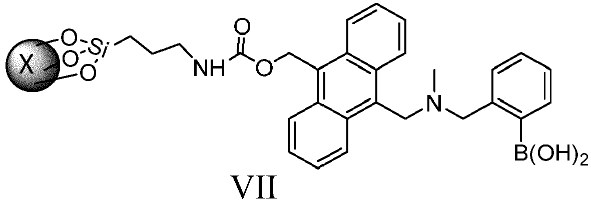 or 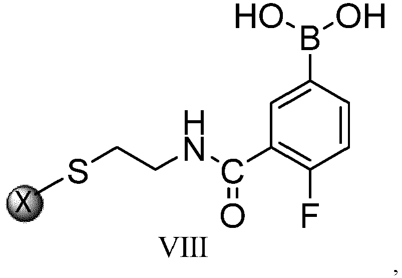 wherein X refers to the nanoparticle pore blocking agent.

The present disclosure further provides a method for preparing the mesoporous silica nanomaterial for controlled release according to any one of the embodiments of the present disclosure, wherein the method comprises the following steps:
(1) providing the mesoporous silica nanoparticles functionalized by a polyhydroxy compound and the pore blocking agents functionalized by phenylboronic acid; and
(2) dispersing the mesoporous silica nanoparticles functionalized by a polyhydroxy compound and the pore blocking agent functionalized by phenylboronic acid in a solvent, and reacting to obtain the mesoporous silica nanomaterial for controlled release.

In one or more embodiments, the mesoporous silica nanomaterial for controlled release further comprises functionally active molecules.

The present disclosure further provides a functionally active molecule nano-drug delivery system, which includes the mesoporous silica nanomaterial for controlled release according to any embodiment of the present disclosure and functionally active molecules.

In one or more embodiments, the size of the functionally active molecule is smaller than the mesopore size of the mesoporous silica nanoparticles.

In one or more embodiments, the functionally active molecule comprises a diabetes therapeutic agent.

In one or more embodiments, the diabetes therapeutic agent is insulin or its biologically active analogues.

In one or more embodiments, the insulin or its biologically active analogues is selected from a group consisting of human insulin, recombinant human insulin, insulin from non-human animals, rapid-acting insulin, rapid-acting insulin analogs, intermediate-acting insulin and long-acting insulin.

In one or more embodiments, the functionally active molecule is loaded inside the mesoporous silica nanoparticles, for example, in the mesopores of the mesoporous silica nanoparticles.

In one or more embodiments, the functionally active molecules include small molecule detection or diagnostic reagents, such as dyes, drugs, indicators, and the like.

In one or more embodiments, the functionally active molecule is functional quantum dots or nanoparticles with a size smaller than the mesopore size of mesoporous silica nanoparticles.

The present disclosure further provides a method for preparing the functionally active molecule nano-drug delivery system according to any embodiment of the present disclosure, and the method comprises the following steps:
(1) providing the mesoporous silica nanoparticles functionalized by a polyhydroxy compound and the pore blocking agents functionalized by phenylboronic acid;
(2) providing the mesoporous silica nanoparticles functionalized by a polyhydroxy compound in a solvent containing functionally active molecules, loading the mesoporous silica nanoparticles with the functionally active molecules; and
(3) adding the pore blocking agent functionalized by phenylboronic acid to the reaction solution in step (2) for reaction to obtain the functionally active molecule nano-drug delivery system.

The present disclosure further provides a kit, wherein the kit comprises mesoporous silica nanoparticles functionalized by a polyhydroxy compound and a nanoparticle pore blocking agent functionalized by phenylboronic acid; preferably, the mesoporous silica nanoparticle functionalized by a polyhydroxy compound are as described in any embodiment of the present disclosure; preferably, the nanoparticle pore blocking agent functionalized by phenylboronic acid is as described in any embodiment of the present disclosure; optionally, the kit further comprises a functionally active molecule; preferably, the functionally active molecule is as described in any embodiment of the present disclosure; preferably, the mesoporous silica nanoparticle functionalized by a polyhydroxy compound, the nanoparticle pore blocking agent functionalized by phenylboronic acid, and the optional functionally active molecule contained in the kit are respectively stored in different containers.

The present disclosure further provides a microneedle or a microneedle array patch, and the microneedle or the microneedle array patch comprises the mesoporous silica nanomaterial for controlled release as described in any embodiment of the present disclosure or the functionally active molecule nano-drug delivery system as described in any embodiment of the present disclosure.

The present disclosure further provides use of the mesoporous silica nanomaterial for controlled release as described in any embodiment of the present disclosure in the preparation of a medicine for treating diabetes or controlling blood sugar levels in a patient.

The present disclosure further provides use of the functionally active molecule nano-drug delivery system as decribed in any embodiment of the present disclosure in the manufacture of a medicine for treating diabetes or controlling blood sugar levels in a patient, wherein the functionally active molecule comprises a diabetes therapeutic agent; preferably, the diabetes therapeutic agent is insulin or its biologically active analogues; preferably, the insulin or its biologically active analogue is selected from a group consisting of human insulin, recombinant human insulin, insulin from non-human animals, rapid-acting insulin, rapid-acting insulin analogs, intermediate-acting insulin and long-acting insulin.

The present disclosure further provides use of the mesoporous silica nanomaterial for controlled release as described in any embodiment of the present disclosure or the functionally active molecule nano-drug delivery system as described in any embodiment of the present disclosure in the preparation of a medicine or a diagnostic reagent or a diagnostic kit for controlled release.

### Brief Description of Figures

Figure 1 is a transmission electron micrograph of the nano-zinc oxide with a size of about 10nm in Example 1.
Figure 2 is infrared spectra of ZnO-P1, mercaptophenylboronic acid, and nano-zinc oxide in Example 2.
Figure 3 is a transmission electron microscope image of mesoporous silica nanoparticles (M0-3) with a size of about 150 nm and uniform and ordered pore channels in Example 5.
Figure 4 is a transmission electron micrograph of the mesoporous silica nanoparticles (M1-1) loaded with fluorescein-labeled insulin and zinc oxide blocked in Example 8.
Figure 5 shows the relationship between the release of FITC-insulin and time of sugar solutions of M1-1 of Example 29 with different concentrations.
Figure 6 shows the relationship between the release of FITC-insulin and time of sugar solutions of M2-1 of Example 30 with different concentrations.
Figure 7 shows the relationship between the release of FITC-insulin and time of sugar solutions of M3-1 of Example 31 with different concentrations.
Figure 8 shows the relationship between the release of FITC-insulin and time of sugar solutions of M4-1 of Example 32 with different concentrations.
Figure 9 shows the cell survival rate of the cells of Example 33 cultured in the medium containing M0-3, M0-4 and M1-1.
Figure 10 is a scanning electron microscope image of the SGRM microneedle patch of Example 35.
Figure 11 is a scanning electron microscope image of the microneedles on the SGRM microneedle patch of Example 35.
Figure 12 shows the changes in blood sugar concentration of the diabetic rats over time after the diabetic rats in Example 37 are injected with 1.5 mg M3-2, 2.0 mg M3-2 (4.1 IU), 1.0 mL PBS buffer solution and insulin (4.1 IU) respectively.
Figure 13 shows the changes in blood sugar concentration of the diabetics rats and the normal rats over time, wherein the diabetic rats in Example 38 are injected with 2.0 mg M3-2 (4.1 IU) and insulin (4.1 IU) respectively, and 2.5h later the diabetic rats and the normal rats are injected with glucose (1.5 g/kg).
Figure 14 shows the changes in blood sugar concentration of the normal rats over time after the normal rats in Example 39 are injected with 2.0 mg M3-2 (4.1 IU) and insulin (4.1 IU) respectively.
Figure 15 shows the changes in blood sugar concentration of the diabetic mice over time after the diabetic mice in Example 40 are pasted with a blank patch, injected with insulin (12.0 IU/kg) and patched with a SGRM microneedle patch (12.0 IU/kg) respectively.
Figure 16 shows the changes in blood sugar concentration of the diabetics mice and the normal mice over time, wherein the diabetic mice in Example 41 are injected with insulin (12.0 IU) and pasted with a SGRM microneedle patch (12.0 IU/kg), and 3h later the diabetic mice and normal mice are injected with glucose (1.5g/kg).
Figure 17 shows the changes in blood sugar concentration of the normal mice over time after the normal mice in Example 42 are pasted with a SGRM microneedle patch (12.0 IU/kg) and injected with insulin (9.5 IU/kg) respectively.
Figure 18 is a transmission electron microscope image of mesoporous silicon oxide nanoparticles with a size of about 170 nm.
Figure 19 is a transmission electron microscope image of mesoporous silicon oxide nanoparticles with a size of about 300 nm.

### Detailed Description

In order to enable those skilled in the art to understand the features and effects of the present disclosure, the following is a general description and definition of terms and words mentioned in the specification and claims. Unless otherwise specified, all technical and scientific terms used herein are intended to be the ordinary meaning of the knowledge of the present disclosure by those skilled in the art, and in case of a conflict, the definition of this specification shall prevail.

The theories or mechanisms described and disclosed As used herein, whether right or wrong, should not limit the scope of the present disclosure in any way, that is to say the present disclosure can be practiced without limitation to any particular theory or mechanism.

The numerical ranges described herein should be considered to encompass and specifically disclose all possible subranges and any individual numerical value within that range. For example, "containing 1 to 20 carbon atoms" will include containing 1 to 10 carbon atoms, containing 2 to 10 carbon atoms, containing 5 carbon atoms, and the like.

As disclosed herein, for the sake of brevity of description, all possible combinations of various technical features in the various embodiments or examples are not described. Therefore, as long as there is no contradiction in the combination of these technical features, the various technical features in the various embodiments or examples can be combined in any combination, and all possible combinations should be considered to be within the scope of this specification.

As used herein, halogen includes F, Cl, Br and I.

As used herein, alkyl refers to a linear or branched monovalent saturated hydrocarbon group, usually containing 1-12 carbon atoms (C₁₋₁₂ alkyl), preferably containing 1-6 carbon atoms (C₁₋₆ alkyl). Examples of alkyl groups include, but are not limited to, methyl, ethyl, n-propyl, isopropyl, n-butyl, isobutyl, tert-butyl, and the like.

As used herein, alkylene refers to a straight or branched divalent saturated hydrocarbon group, usually containing 1-12 carbon atoms (C₁₋₁₂ alkylene), preferably containing 1-6 carbon atoms (C₁₋₆ alkylene). Examples of alkylene groups include, but are not limited to, methylene, ethylene, propylene, and the like.

As used herein, alkenyl refers to a group having at least one double bond in a straight or branched chain, and its carbon chain length is usually 2-12 carbon atoms, preferably 2-6 carbon atoms. Typical alkenyl groups include vinyl, 1-propenyl, 2-propenyl, 2-methyl-1-propenyl, 1-butenyl and 2-butenyl. Alkenylene refers to a straight-chain or branched divalent alkenyl group, such as vinylene (-CH=CH-).

As used herein, alkynyl refers to a group having at least one triple bond in a straight or branched chain, and its carbon chain length is usually 2-12 carbon atoms, preferably 2-6 carbon atoms. Typical alkynyl groups include ethynyl, 1-propynyl, 1-methyl-2-propynyl, 2-propynyl, 1-butynyl and 2-butynyl.

As used herein, alkoxy refers to "alkyl-O-", wherein the alkyl group may have 1-12 carbon atoms, preferably 1-6 carbon atoms.

As used herein, electron-withdrawing group substituents may be selected from a group consisting of nitro, cyano, halogen, carboxyl, alkynyl, and alkenyl; electron-donating group substituents may be selected from a group consisting of alkyl, secondary amine, primary amine, tertiary amine, hydroxyl and alkoxy.

As used herein, a reactive functional group refers to a functional group that can undergo reactions such as addition, condensation, cyclization, and polymerization for covalently connecting, including but not limited to: amino group, hydroxyl group, carboxyl group, sulfonic acid group, mercapto group, alkenyl group, alkynyl group, azido group, tetrazine structure, halogen, hydrazine, epoxy group, isocyanate group, isothiocyanate group, etc.

As used herein, a derivative refers to a relatively complex product derived from the substitution of hydrogen atoms or atomic groups in a compound by other atoms or atomic groups.

As used herein, silicon dioxide and silica have the same meaning.

The mesoporous silica nanomaterials for controlled release (hereinafter referred to as controlled release nanomaterials) of the present disclosure contain mesoporous silica nanoparticles functionalized by a polyhydroxy compound and a nanoparticle pore blocking agent functionalized by phenylboronic acid. In certain embodiments, the controlled release nanomaterials of the present disclosure consist of mesoporous silica nanoparticles functionalized by a polyhydroxy compound and a nanoparticle pore blocking agent functionalized by phenylboronic acid. In the present disclosure, the nanoparticle pore blocking agent functionalized by phenylboronic acid can be connected to the mesoporous silica nanoparticles functionalized by a polyhydroxy compound through the phenylboronic acid ester bond formed between the phenylboronic acid functional group and the polyhydroxyl functional group, thereby blocking the mesoporous openings of mesoporous silica nanoparticles.

In the present disclosure, the mesoporous silica nanoparticles can be various biocompatible or non-biocompatible mesoporous silica nanoparticles known in the art, preferably biocompatible mesoporous silica nanoparticles. Its particle size is between 90-350 nm, and its pore size is between 5-20 nm. Mesoporous silica nanoparticles are usually prepared by a hydrothermal method. For example, cationic surfactants (such as cetyltrimethylammonium bromide (CTAB)) as templates, supplemented with a small molecule organic amine (or ammonia) and an organic additive (such as n-hexane, etc.) are used to make it react with tetraethyl orthosilicate under appropriate conditions to form the mesoporous silica nanoparticles of the present disclosure. Therefore, in a preferred embodiment, the mesoporous silica nanoparticles suitable for the present disclosure are the reaction product of a cationic surfactant and tetraethyl orthosilicate, supplemented with a small molecule organic amine (or ammonia) and an organic additive, more preferably the reaction product of CTAB and tetraethyl orthosilicate, supplemented with ammonia and n-hexane. Usually, CTAB, tetraethyl orthosilicate, ammonia and n-hexane can be reacted in, for example, pure water at about 35° C for about 12 hours, to obtain the mesoporous silica nanoparticles of the present disclosure. Methyl orthosilicate, propyl orthosilicate, etc. may be used instead of ethyl orthosilicate.

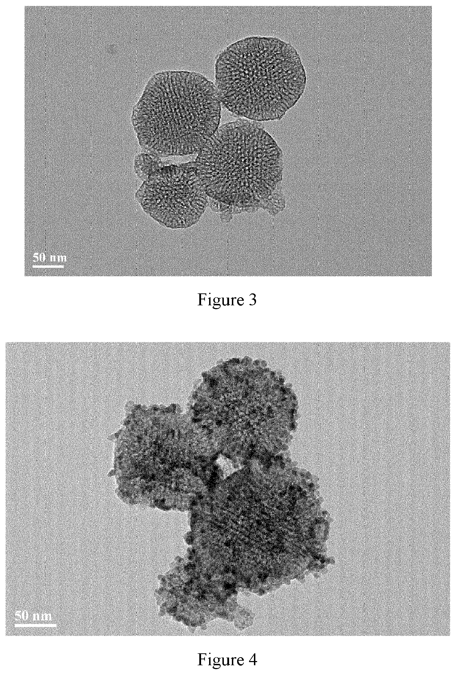

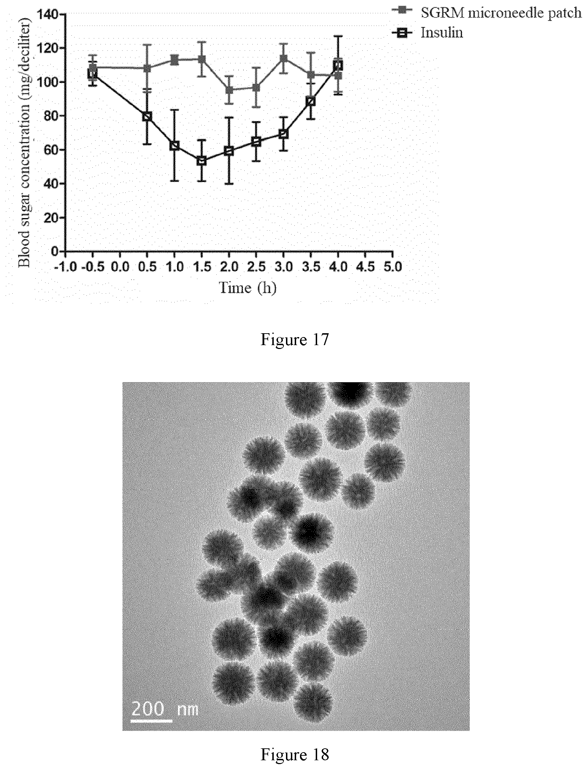

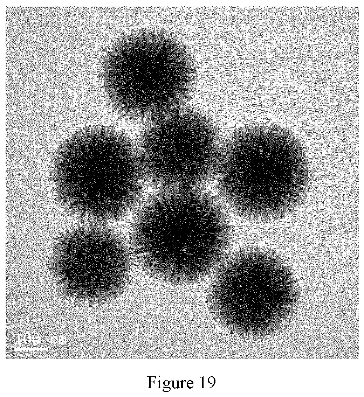

The particle size and pore size of the mesoporous silica nanoparticles can be controlled by controlling the reaction conditions and the like. The pore size of the mesoporous silica nanoparticles of the present disclosure is preferably between 5-20 nm, for example, between 5-10 nm, 10-20 nm, 10-15 nm, etc. The particle size of the mesoporous silica nanoparticles is preferably in the range of 90-350 nm, for example, 100-150 nm, 100-300 nm (as shown in Figure 18), 200-300 nm (such as shown in Figure 19), etc. There are no corresponding restrictions on pore size and particle size, for example, the mesopore size corresponding to a particle size of 100-150 nm can be 5-10 nm (as shown in Figure 3), or 8-12 nm; the size corresponding to 200-300 nm can be 5-10 nm, or 10-20 nm, etc. The shape of the pore channels can be radial, ordered mesoporous, random, etc.

As used herein, the mesoporous silica nanoparticles functionalized by a polyhydroxy compound mean that there are reactive polyhydroxy functional groups on the surface of the mesoporous silica nanoparticles, such as 1,2-dihydroxy functional groups. The reactivity means that the polyhydroxy functional group can react with the phenylboronic acid functional group.

The polyhydroxy compound suitable for the present disclosure generally has the following structure: R₁-R₂ , wherein R₁ is a group that can react with the linker molecule described below, including but not limited to amino and carboxyl, and R₂ is a straight or branched C₂₋₁₂ alkyl, C₂₋₁₂ alkenyl or C₂₋₁₂ alkynyl, wherein the alkyl, alkenyl and alkynyl are substituted by at least 2 hydroxyl groups, and at least two adjacent C atoms are each substituted by 1 hydroxyl group. Exemplary polyhydroxy compounds include but are not limited to, carbohydrate molecules such as monosaccharides and their derivatives, polysaccharides and their derivatives, and also include synthetic block copolymers containing 2 or more hydroxyls. In certain embodiments, the polyhydroxy compound of the present disclosure is selected from a group consisting of monosaccharides and their derivatives, including but not limited to glucose and its derivatives, fructose and its derivatives, mannose and its derivatives, and galactose and its derivatives. Typically, open-chain structures of these carbohydrate molecules are used. The "derivatives" mentioned herein refer to derivatives that retain two or more hydroxyl groups, especially including sugar acid, which is an acid formed by oxidizing the aldehyde group in the open-chain sugar molecule to a carboxyl group under the action of a weak oxidant or enzyme. In certain embodiments, the polyhydroxy compound used in the present disclosure is selected from a group consisting of open-chain glucose and its derivatives, such as gluconic acid.

A polyhydroxy compound can be attached to mesoporous silica nanoparticles through a linker molecule, which generally has both a polyhydroxy compound-reactive group and a mesoporous silica nanoparticle-reactive group. For example, the linker molecule is typically a molecule comprising trialkoxysilane moiety, such as aminoalkyltrialkoxysilane, wherein the group capable of reacting with mesoporous silica nanoparticles is a trialkoxy silane group. Usually, the connection mode of the polyhydroxy compound and the linker molecule may be an amide group (-NH-CO-). In certain embodiments, the linker molecule is a silane coupling agent having a group capable of reacting with the polyhydroxy compound, such as 3-isocyanatopropyltrimethoxysilane, 3-aminopropyltrimethoxysilane, etc. In some preferred embodiments, the linker moiety is aminopropyltriethoxysilane or aminopropyltrimethoxysilane. The linker molecule can be reacted with mesoporous silica nanoparticles first to obtain mesoporous silica nanoparticles having a group capable of reacting with the polyhydroxy compound, which is then reacted with the polyhydroxy compound to obtain mesoporous silica nanoparticles functionalized by the polyhydroxy compound.

It is also possible to first react the linker molecule with the polyhydroxy compound to obtain a polyhydroxy compound having a group capable of reacting with mesoporous silica nanoparticles, which is then reacted with mesoporous silica nanoparticles to obtain mesoporous silica nanoparticles functionalized by the polyhydroxy compound. For example, in certain embodiments, 3-aminopropyltrimethoxysilane is first reacted with gluconic acid to obtain N-(3-trimethoxysilylpropyl)glucamide, and then N-(3-trimethoxysilylpropyl)glucamide is reacted with mesoporous silica to obtain mesoporous silica nanoparticles functionalized by the polyhydroxy compound.

The polyhydroxy compound can also be a commercially available polyhydroxy compounds having groups capable of reacting with mesoporous silica nanoparticles, such as trialkoxysilane groups, such as N-(3-trimethoxysilane propyl) glucamide. That is, in certain embodiments, the polyhydroxy compound can be directly connected to the mesoporous silica nanoparticles. In these embodiments, the polyhydroxy compound may also be considered to already comprise a linker molecule.

In certain embodiments, the mesoporous silica nanoparticles functionalized by the polyhydroxy compound have a structure shown in the following formula I: 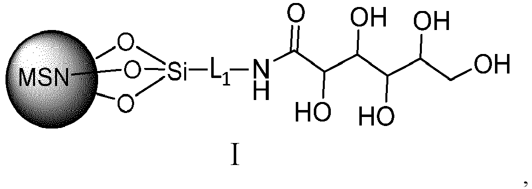 wherein MSN refers to mesoporous silica nanoparticles; L₁ is a linker group.

Those skilled in the art can understand that, in the present disclosure, the linker group L₁ is not particularly limited, as long as it is a group that can firmly connect the polyhydroxy compound to the mesoporous silica nanoparticles, and the firmness means that the linking group will not be broken under the action of the competitive substrate (monosaccharide compounds such as glucose and fructose, oligosaccharides, polysaccharides, and catechol-containing compounds such as catechol) of phenylboronate. The linker group L₁ can be considered as a part of the linker molecule connecting the mesoporous silica nanoparticles and the polyhydroxy compound as described above, or a part of the polyhydroxy compound having a group capable of reacting with the mesoporous silica nanoparticles, or a combination of the part of the linker molecule connecting the mesoporous silica nanoparticles and the polyhydroxy compound as described above and the part of the polyhydroxy compound.

In certain embodiments, L₁ is C₁₋₆ alkylene, such as propylene (-CH₂CH₂CH₂-).

In certain embodiments, the mesoporous silica nanoparticle functionalized by the polyhydroxy compound is:

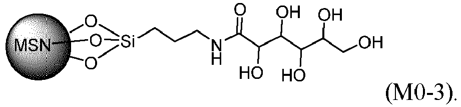

The nanoparticle pore blocking agent suitable for the present disclosure can be various nanoparticles that match the mesopore size of the mesoporous silica nanoparticles, including but not limited to metal oxide nanoparticles, metal sulfide nanoparticles, metal nanoparticles, quantum dots, block copolymers, natural polymers and biomacromolecules. For example, it can be zinc oxide nanoparticles, zinc sulfide nanoparticles, zinc sulfide-zinc oxide core-shell nanoparticles, manganese zinc sulfide nanoparticles, cadmium selenide, ferroferric oxide, gold nanoparticles, and the like. In certain embodiments, the nanoparticle pore blocking agents used in the present disclosure are metal oxide nanoparticles, such as zinc oxide nanoparticles.

It can be understood by those skilled in the art that the size of the nanoparticle pore blocking agent should match the mesopore size of the mesoporous silica nanoparticles. That is, the size of the nanoparticle pore blocking agent can be slightly smaller than, approximately equal to or slightly larger than the mesopore size of the mesoporous silica nanoparticles. For example, the size of the nanoparticle pore blocking agent can be 3-25 nm, such as 3-15 nm, 8-25 nm, 8-20 nm and so on.

After the nanoparticle pore blocking agent is functionalized by phenylboronic acid, it can be connected to the mesoporous silica nanoparticles functionalized by the polyhydroxy compound through phenylboronic acid ester structure, thereby effectively blocking the mesoporous openings of the mesoporous silica nanoparticles and preventing the release of internal loads; at the same time, the nanoparticle pore blocking agent can be used as a contrast agent, a photothermal therapy agent or an additional source of some special elements of a living body.

In the present disclosure, the phenylboronic acid compound refers to a small molecular compound used to functionalize the nanoparticle pore blocking agent by phenylboronic acid, which has a phenylboronic acid group, and usually has one or more reactive functional groups other than the phenylboronic acid group. Phenylboronic acid group is capable of reacting with polyhydroxy functional groups such as 1,2-dihydroxy functional group to form phenylboronic ester linkages.

In certain embodiments, the phenylboronic acid compound has a reactive functional group (such as hydroxyl, amino, carboxyl, etc.) capable of reacting with the linker molecule, and can be connected to nanoparticles of the pore blocking agent through the linker molecule, and the linker molecule has both a group capable of reacting with the phenylboronic acid compound and a group capable of reacting with the nanoparticle pore blocking agent. For example, these linker molecules are typically molecules comprising trialkoxysilane moieties, such as aminoalkyltrialkoxysilanes, wherein the group capable of reacting with nanoparticle pore blocking agent is a trialkoxy silane group. The group capable of reacting with the phenylboronic acid compound may be, for example, the reactive functional groups described herein (e.g., amino group, isocyanate group, etc.). Usually, the connection mode between the phenylboronic acid compound and the linker molecule may be an amide group (-NH-CO-). In certain embodiments, the linker molecule is 3-isocyanatopropyltrimethoxysilane or 3-aminopropyltriethoxysilane.

In certain embodiments, the phenylboronic acid compound has a group capable of reacting with the nanoparticle pore blocking agent (e.g., a trialkoxysilane group or a mercapto group), which can be directly connected to the nanoparticle pore blocking agent. In these embodiments, the phenylboronic acid compound may be considered to have already included the linker molecule. For example, the phenylboronic acid compound having a group capable of reacting with the nanoparticle pore blocking agent may be the following phenylboronic acid compound having a mercapto group:

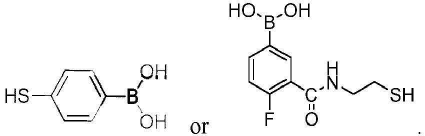

In some embodiments, the phenylboronic acid compound is selected from a structure shown in the following formula (A): 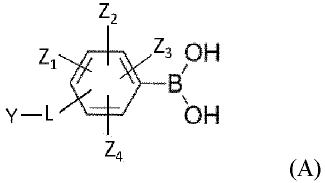 wherein:
Z₁, Z₂, Z₃ and Z₄ are each independently selected from a group consisting of H, an electron-withdrawing group substituent or an electron-donating group substituent, preferably each independently selected from a group consisting of hydrogen, C₁₋₆ alkyl, halogen, nitro, carboxyl and amino, more preferably each independently selected from a group consisting of hydrogen, C₁₋₆ alkyl and halogen (such as fluoro);
L is absent, or is a linker group connecting the benzene ring and Y, preferably, the linker group is selected from a group consisting of: C₁₋₆ alkylene, -(CH₂)ₙ-NR'-CO-aryl-N=N-aryl-NR'-(CH₂)ₒ-, -(CH₂)ₙ-NR'-COO-(CH₂)ₘ-aryl-(CH₂)ₒ-NR'-(CH₂)ₚ-, -(CH₂)ₙ-NR'-CO-, -(CH₂)ₙ-CO-NR'-, -(CH₂)ₙ-NR'-aryl-(CH₂)ₘ-, -(CH₂)ₙ-NR'-(CH₂)ₘ-aryl-(CH₂)ₒ-, -(CH₂)ₙ-NR'-(CH₂)ₘ-aryl-N=N-aryl-(CH₂)ₒ- and -C₂₋₆ alkenylene-aryl-(CH₂)ₒ-; wherein n, m, o and p are each independently an integer of 0-4; R' is H or C₁₋₄ alkyl; the aryl is selected from a group consisting of phenyl, naphthyl, anthracenyl and phenanthrenyl, and may be optionally substituted by 1-3 substituents selected from a group consisting of halogen, C₁₋₄ alkyl and C₁₋₄ alkoxy; and
Y is a group reacting with the linker molecule on the nanoparticle pore blocking agents, selected from a group consisting of amino, hydroxyl, carboxyl, sulfonic acid, mercapto, alkenyl, alkynyl, azido, tetrazine, halogen, hydrazine, epoxy, isocyanate and isothiocyanate; preferably hydroxyl, amino, carboxyl, sulfonic acid or mercapto.

In some preferred embodiments, in the structure of formula A, Z₁, Z₂, Z₃ and Z₄ are each independently selected from a group consisting of hydrogen, C₁₋₆ alkyl and halogen; L is absent or is: C₁₋₆ alkylene, -(CH₂)ₙ-NR'-phenyl-N=N-phenyl-, -(CH₂)ₙ-NR'-(CH₂)ₘ-aryl-(CH₂)ₒ- , or -C(O)-NH-C₁₋₆ alkyl-, wherein n, m and o are each independently an integer of 0-4, R' is hydrogen or C₁₋₄ alkyl, and the phenyl or aryl may be optionally substituted by 1-3 substituents selected from C₁₋₄ alkyl, and the aryl is phenyl, naphthyl or anthracenyl.

In certain embodiments, the phenylboronic acid compound may be selected from the following structures: 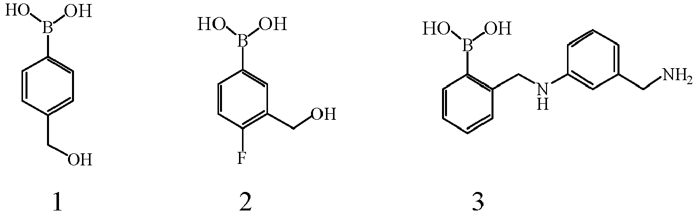 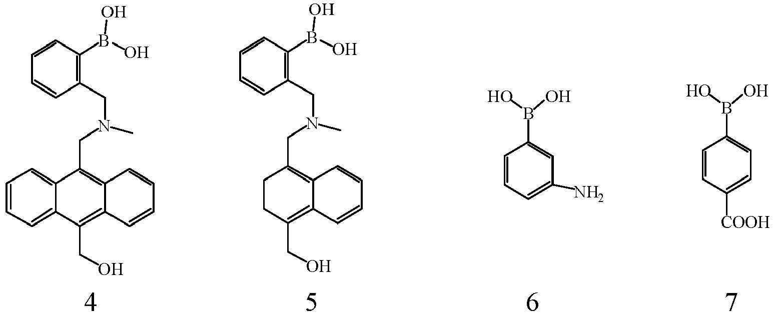 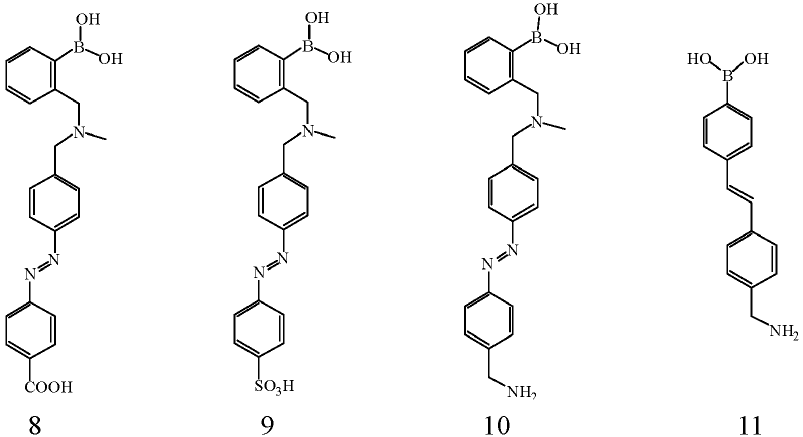 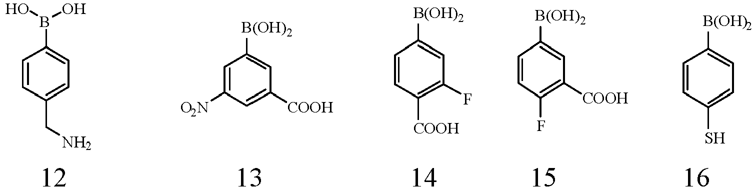 and

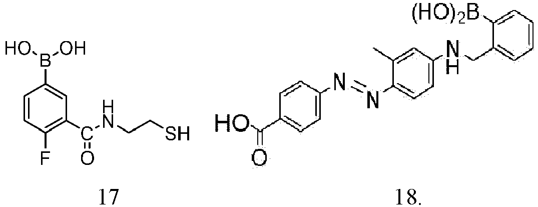

In the present disclosure, the nanoparticle pore blocking agent functionalized by phenylboronic acid means that there is a phenylboronic acid functional group having a reactivity on the surface of the nanoparticle pore blocking agent, and the reactivity means that the phenylboronic acid functional group can react with the polyhydroxy functional group.

The nanoparticle pore blocking agent functionalized by phenylboronic acid can be obtained by directly reacting the nanoparticle pore blocking agent with a phenylboronic acid compound having a group capable of reacting with a nanoparticle pore blocking agent, and can also be obtained by reacting the nanoparticle pore blocking agent with a linker molecule to obtain a surface-functionalized nanoparticle pore blocking agent (that is, having a group capable of reacting with a phenylboronic acid ester compound), and then reacting with the phenylboronic acid ester compound, and can also be obtained by reacting a phenylboronic acid ester compound with a linker molecule to obtain a molecule capable of reacting with a nanoparticle pore blocking agent, and then reacting with the nanoparticle pore blocking agent.

The phenylboronic acid compound having a group capable of reacting with the nanoparticle pore blocking agent may be a commercially available phenylboronic acid ester compound, or a compound obtained by reacting a phenylboronic acid ester compound with a linker molecule.

In some embodiments, the nanoparticle pore blocking agent functionalized by phenylboronic acid has a structure shown in the following formula III or IV: 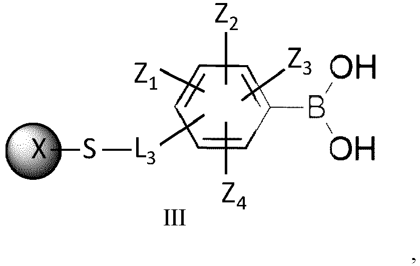 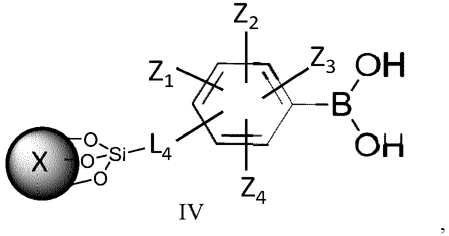 wherein X refers to the nanoparticle pore blocking agent; Z₁, Z₂, Z₃ and Z₄ are each independently selected from a group consisting of hydrogen, electron-withdrawing group substituents and electron-donating group substituents, preferably each independently selected from a group consisting of hydrogen, C₁₋₆ alkyl, halogen, nitro, carboxyl and amino, more preferably each independently selected from a group consisting of hydrogen and halogen (such as fluoro); L₃ and L₄ are each independently absent or a linker group; preferably, the linker group is selected from a group consisting of: C₁₋₆ alkylene, -(CH₂)ₙ-NR'-CO-aryl-N=N-aryl-NR'-(CH₂)ₒ-, -(CH₂)ₙ-NR'-COO-(CH₂)ₘ-aryl-(CH₂)ₒ-NR'-(CH₂)ₚ-, -(CH₂)ₙ-NR'-CO-, -(CH₂)ₙ-CO-NR'-, -(CH₂)ₙ-NR'-aryl-(CH₂)ₘ-, -(CH₂)ₙ-NR'-(CH₂)ₘ-aryl-(CH₂)ₒ-, -(CH₂)ₙ-NR'-(CH₂)ₘ-aryl-N=N-aryl-(CH₂)ₒ- and -C₂₋₆ alkenylene-aryl-(CH₂)ₒ-; wherein n, m, o and p are each independently an integer of 0-4; R' is H or C₁₋₄ alkyl; the aryl is selected from a group consisting of phenyl, naphthyl, anthracenyl and phenanthrenyl and can be optionally substituted by 1-3 substituents selected from a group consisting of halogen, C₁₋₄ alkyl and C1-4 alkoxy.

Preferably, L₃ and L₄ are each independently absent, or each independently selected from a group consisting of: -(CH₂)ₙ-NR'-CO-aryl-N=N-aryl-NR'-(CH₂)ₒ-(such as -(CH₂)ₙ-NR'-CO-phenyl-N=N-phenyl-NR'-(CH₂)ₒ-), -(CH₂)ₙ-NR'-COO-(CH₂)ₘ-aryl -(CH₂)ₒ-NR'-(CH₂)ₚ- and -amido-C₁₋₆ alkylene-; wherein n, m, o and p are each independently an integer of 0-4; R' is H or C₁₋₄ alkyl; the aryl is selected from a group consisting of phenyl, naphthyl, anthracenyl and phenanthrenyl, and can be optionally substituted by 1-3 substituents selected from a group consisting of halogen, C₁₋₄ alkyl and C₁₋₄ alkoxy.

Those skilled in the art can understand that, in the present disclosure, L₃ and L₄, as a linker group, are not particularly limited, as long as it is a group that can firmly connect the polyhydroxy compound and the mesoporous silica nanoparticles, and the firmness means that the linking group will not be broken under the action of the competitive substrate (monosaccharide compounds such as glucose and fructose, oligosaccharides, polysaccharides, and catechol-containing compounds such as catechol) of phenylboronic acid ester. Exemplary L₃ and L₄ may be as described above in the definition of L in formula A.

In formulas III and IV, the relative positions of Z₁, Z₂, Z₃, Z₄, L₃, L₄ and the phenylboronic acid group on the benzene ring are not limited, that is, L₃ or L₄ may be located in meta, para or ortho position of the phenylboronic acid group.

In some embodiments, the nanoparticle pore blocking agent functionalized by phenylboronic acid is: 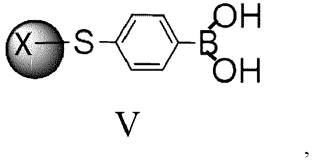 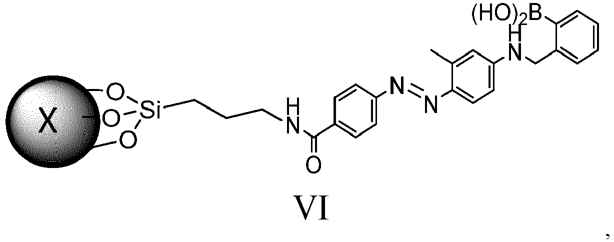 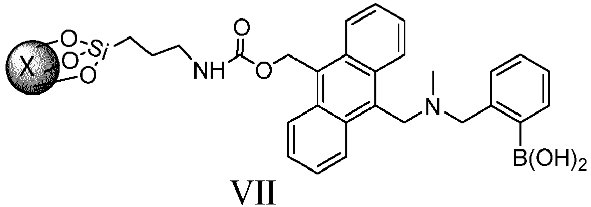 or 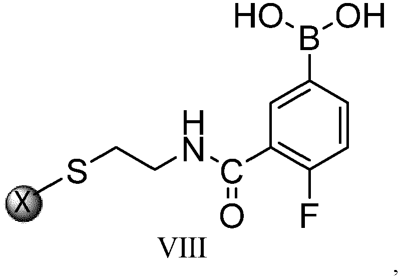 wherein X refers to the nanoparticle pore blocking agent.

The mesoporous silica nanomaterial for controlled release of the present disclosure can be prepared by the following steps:
(1) providing mesoporous silica nanoparticles functionalized by a polyhydroxy compound and a pore blocking agent functionalized by phenylboronic acid; and
(2) dispersing the mesoporous silica nanoparticles functionalized by the polyhydroxy compound and the pore blocking agent functionalized by phenylboronic acid in a solvent, and reacting to obtain the mesoporous silica nanomaterial for controlled release.

The mesoporous silica nanoparticles functionalized by the polyhydroxy compound and the pore blocking agent functionalized by phenylboronic acid can be prepared by the method described as used herein, and the specific reaction conditions can be determined according to the actual reaction.

The reaction in step (2) is carried out under the condition that the pore blocking agent is allowed to block the mesopores. For example, the reaction of step (2) can be carried out at a temperature lower than 40°C (such as room temperature), and the solvent used is not particularly limited, and usually may be one of toluene, dimethylformamide, dimethylacetamide, dimethylsulfoxide (DMSO), dichloromethane, ethanol, methanol, water, various buffers based on aqueous solutions or mixtures thereof. For example, the solvent may be a mixture of phosphate buffered saline (PBS buffer) and DMSO.

The substrate suitable for the present disclosure can be various substrates that can be combined with phenylboronic acid, and the binding force of the substrate with phenylboronic acid is not less than the binding force of the substrate with polyhydroxy compounds that form esters with phenylboronic acid, so that the substrate can break the phenylboronic acid ester bond, thereby releasing the nanoparticle pore blocking agent, and finally opening the mesoporous channel to release the internal load (such as various types of insulin, dyes, drugs, indicators or other biological macromolecules, etc.).

Preferably, the substrate used is biocompatible. For example, the substrate itself is carried by human or animal body itself, such as those occur naturally in blood (such as glucose, catechol, etc.), or those are artificially administered (such as fructose, other types of monosaccharides, oligosaccharides, or polysaccharides, etc.).

Therefore, in order to make the controlled release nanomaterials of the present disclosure biocompatible, the surface of the mesoporous silica nanoparticles and the nanoparticle pore blocking agent may be further modified to increase its biological safety, such as coating a layer of PEG material, pH-responsive medical polymer material or other biodegradable material on the outer surface.

The controlled release nanomaterials of the present disclosure may also contain a composition or a mixture of other ingredients such as pharmaceutically acceptable carriers or excipients. Those skilled in the art can select appropriate pharmaceutically acceptable carriers or excipients according to the use of the controlled release nanomaterial of the present disclosure.

In addition to the above-mentioned mesoporous silica nanoparticles functionalized by a polyhydroxy compound and the above-mentioned nanoparticle pore blocking agent functionalized by phenylboronic acid, the controlled release nanomaterial of the present disclosure may also contain functionally active molecules. In certain embodiments, the controlled release nanomaterial of the present disclosure consists of mesoporous silica nanoparticles functionalized by a polyhydroxy compound, a nanoparticle pore blocking agent functionalized by phenylboronic acid and functionally active molecules.

The controlled release nanomaterials containing the mesoporous silica nanoparticles functionalized by a polyhydroxy compound, the nanoparticle pore blocking agent functionalized by phenylboronic acid, and functionally active molecules of the present disclosure can realize the loading and controlled release of functionally active molecules. Therefore, the present disclosure is also called functionally active molecule nano-drug delivery system.

It should be understood that the functionally active molecule nano-drug delivery system of the present disclosure refers to a product capable of controlling the loading and controlled release of functionally active molecules (such as insulin). The functionally active molecule nano-drug delivery system of the present disclosure can be a simple product containing only mesoporous silica nanoparticles functionalized by a polyhydroxy compound, a nanoparticle pore blocking agent functionalized by phenylboronic acid, and functionally active molecules, and can also be a composition or mixture comprising mesoporous silica nanoparticles functionalized by a polyhydroxy compound, a nanoparticle pore blocking agent functionalized by phenylboronic acid, functionally active molecules and other ingredients such as pharmaceutically acceptable carriers or excipients. Those skilled in the art can select appropriate pharmaceutically acceptable carriers or excipients according to the use of the composition or mixture.

The controlled release nanomaterial and the functionally active molecule nano-drug delivery system of the present disclosure can be used to control the release of the functionally active molecules. Therefore, the functionally active molecules that can be used in the present disclosure include functionally active molecules for various purposes, for example, biomacromolecules such as proteins and nucleic acids, small molecular compounds such as antitumor drugs and fluorescent probes, and various functional quantum dots or nanoparticles having a size smaller than the mesopore size of mesoporous silica nanoparticles. As used herein, small molecules and small molecule compounds refer to various compounds that can enter the mesoporous channels of mesoporous silica nanoparticles. Proteins can be, for example, various enzymes and antibodies useful for therapeutic or diagnostic use. Nucleic acid can be, for example, various functional nucleic acids that can be used in gene therapy, such as siRNA. The functionally active molecules loaded in the controlled release nanomaterial and the functionally active molecule nano-drug delivery system of the present disclosure can be one or a combination of more of the above-mentioned functionally active molecules for diagnosis or treatment of the same or different diseases or symptoms.

The size of the functionally active molecule is preferably smaller than the mesopore size of the mesoporous silica nanoparticles. Preferably, the functionally active molecules are loaded inside the mesoporous silica nanoparticles, for example, loaded in the mesoporous channels of the mesoporous silica nanoparticles.

In the present disclosure, the functionally active ingredient preferably contains a diabetes therapeutic agent. In certain embodiments, the functionally active molecule may only contain the diabetes therapeutic agent, or may consist of the diabetes therapeutic agent and other small molecule detective or diagnostic reagents (such as dyes, drugs, indicators, etc.). The diabetes therapeutic agent suitable for use in the present disclosure is preferably insulin or a biologically active analogue thereof.

Preferably, the insulin or its biologically active analogues is selected from a group consisting of human insulin, recombinant human insulin, insulin from non-human animals, rapid-acting insulin, rapid-acting insulin analogs, intermediate-acting insulin and long-acting insulin. Insulins suitable for the present disclosure include but are not limited to insulin monomers, dimers of insulin, hexamers or other aggregated forms of insulin, modified insulins, labeled insulins (such as fluorescein-labeled insulins) or other insulin substitutes.

The functionally active molecular nano-drug delivery system of the present disclosure can be prepared by the following steps:
(1) functionalizing mesoporous silica nanoparticles by a polyhydroxy compound to obtain mesoporous silica nanoparticles functionalized by the polyhydroxy compound having hollow channels;
(2) dispersing the mesoporous silica nanoparticles functionalized by the polyhydroxy compound obtained in step (1) in a solvent containing functionally active molecules, so as to load the mesoporous silica nanoparticles with the functionally active molecules; and
(3) adding a nanoparticle pore blocking agent functionalized by phenylboronic acid to the reaction solution in step (2) for reaction under the condition that the pore blocking agent is allowed to block the mesopores, to obtain the functionally active molecule nano-drug delivery system.

Mesoporous silica nanoparticles can be prepared by a hydrothermal method. An exemplary method of preparing mesoporous silica nanoparticles can be referred to Example 3 of the present disclosure. Various mesoporous silica nanoparticles known in the prior art (such as those are commercially available) can also be used directly.

Methods for functionalizing mesoporous silica nanoparticles are well known in the art, and are illustrated in Example 4 of the present disclosure. Functionalization of mesoporous silica nanoparticles can be carried out by dispersing mesoporous silica nanoparticles in a solvent (such as ethanol), reacting with a polyhydroxy compound, or a compound formed by the reaction of the polyhydroxy compound and a linker molecule, or reacting with the linker molecule first, followed by the polyhydroxy compound; and the conditions of the above reaction may be known in the art.

In some embodiments, there is a surfactant in the mesoporous structure of the mesoporous silica nanoparticles prepared by the hydrothermal method. In order to remove the surfactant so as to load functionally active molecules in the mesoporous structure, step (1) also includes: removing the surfactant after functionalization of the mesoporous silica nanoparticles with the polyhydroxy compound. The method for removing surfactants is well known in the art, and is illustrated in Example 5 of the present disclosure. For example, mesoporous silica nanoparticles may be dispersed in a solvent (such as ethanol), a certain amount of salt solution (such as an aqueous solution of ammonium nitrate) is added for reaction under reflux.

After the mesoporous silica nanoparticles functionalized by the polyhydroxy compound are obtained, they can be contacted with functionally active molecules in a solvent, so that the functionally active molecules can be loaded into the mesoporous silica nanoparticles; further, the mesoporous silica nanoparticles functionalized by the polyhydroxy compound and loaded with functionally active molecules can react with the nanoparticle pore blocking agent functionalized by phenylboronic acid in the solvent, and the mesopore openings are blocked by the intercombination of phenylboronic acid and polyhydroxy compound.

There are no special restrictions on the solvent used in steps (1) to (3), which can be determined according to the actual reaction, and may be usually one of toluene, dimethylformamide, dimethylacetamide, dimethyl sulfoxide, methylene chloride, ethanol, methanol, water, various buffers based on aqueous solution or their mixtures. Likewise, the present disclosure has no special limitations on the amount of reactants, reaction time, temperature and etc. used in steps (1) to (3). A skilled person can select appropriate reaction conditions according to actual preparation conditions. An exemplary preparation can be found in the examples section of this disclosure. In a specific example, the solvent used in step (2) is a phosphate buffer with a pH value of about 7.4, and the reaction is performed below 40° C (e.g., room temperature) protected from light. In a specific example, the solvent used in step (3) is a mixture of phosphate buffer and DMSO with a pH value of about 7.4, and the reaction is performed at a temperature lower than 40° C (e.g., room temperature).

Different methods can be used to test the performance of the controlled release nanomaterial or functionally active molecule nano-drug delivery system of the present disclosure. For example, the functionally active molecule nano-drug delivery system of the present disclosure can be uniformly dispersed in the corresponding test system, and then, after full contact with the corresponding stimulus (substrate concentration), the mesopores are opened, and the internal loads, namely functionally active molecules, are released. According to the present disclosure, fluorescently labeled insulin can be used as the load molecule for characterization, but not limited thereto.

The contact time and the amount of controlled release nanomaterial or functionally active molecular nano-drug delivery system used can be determined by the skilled person according to the actual situation. For example, the contact time and dosage can be determined according to the content of different substrates in the corresponding system and other factors.

After the stimulation source is in contact with the controlled release nanomaterial or functionally active molecule nano-drug delivery system of the present disclosure for a period of time, the mesoporous silica nanoparticles and the test system can be separated by means of centrifugation, etc., and then the ultraviolet absorption intensity in the system or the content of the released internal load, that is, the functionally active molecule, is detected by ultraviolet spectrophotometer or high performance liquid chromatography, so as to determine the release rate and release amount of the loaded molecule.

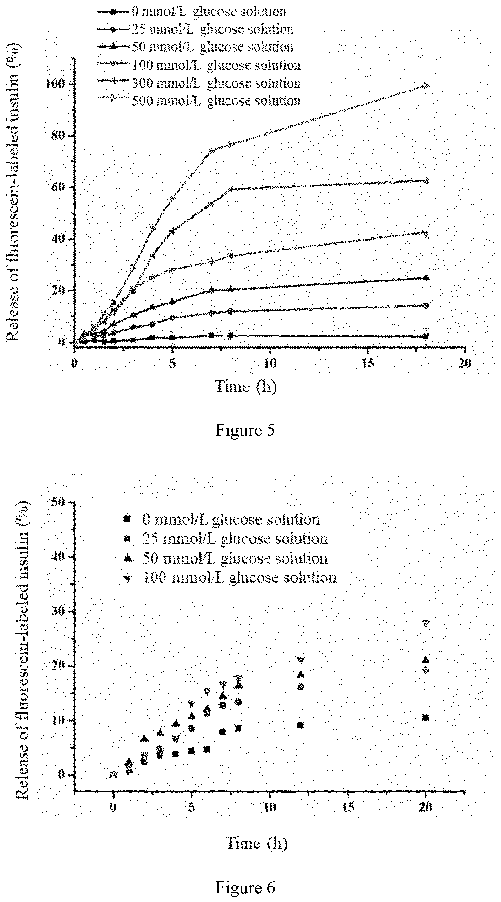

In the present disclosure, different controlled release nanomaterials and functionally active molecule nano-drug delivery systems can be constructed by adjusting the structure of phenylboronic acid ester, so as to realize the response to substrates in different concentration ranges. In some embodiments, the present disclosure selects the phenylboronic acid ester structure that responds to the range of blood sugar concentration, and constructs controlled release nanomaterial and a functionally active molecules nano-drug delivery system based on mesoporous silica, which can realize the real-time controlled release of the internal load as a function of the change of blood sugar concentration (controlled release of insulin as shown in Figures 5-8).

Therefore, based on the controlled release nanomaterials and functionally active molecule nano-drug delivery systems corresponding to different structures of phenylboronic acid ester in the present disclosure, one of them can be used, or two or more can be used simultaneously in practical applications, in order to realize the response to different substrate concentrations at different stages, and finally to control the release rate and release amount of the internal load. When two or more are used at the same time, the usage ratio can be adjusted according to actual conditions.

The controlled release nanomaterial or functionally active molecular nano-drug delivery system of the present disclosure can exist in the form of dry solid powder, or in the form of a solution, or can be loaded on other materials or implanted devices, such as microneedle or microneedle array patch (referred to as microneedle patch). The present disclosure includes a kit, which comprises the mesoporous silica nanoparticles functionalized by the polyhydroxy compound as described in any embodiment of the present disclosure, the nanoparticle pore blocking agent functionalized by phenylboronic acid as described in any embodiment of the present disclosure, and the optional functionally active molecules as described in any embodiment of the present disclosure. Preferably, the mesoporous silica nanoparticles functionalized by polyhydroxy compound, the nanoparticle pore blocking agent functionalized by phenylboronic acid and the optional functionally active molecules comprised in the kit are respectively stored in different containers.

The present disclosure further provides a microneedle or a microneedle array patch, which comprises the mesoporous silica nanomaterial for controlled release as described in any embodiment of the present disclosure or the functionally active molecule nano-drug delivery system as described in any embodiment of the present disclosure.

In the present disclosure, the microneedle and microneedle array patch have meanings known in the art. Generally, a microneedle refers to a tiny needle with a diameter of less than 500 µm (the tip diameter is usually less than 20 µm) and a length of 100-1000 µm. The microneedle array patch usually refers to a tiny patch with microneedles densely distributed on the substrate, and the density of microneedles on the microneedle array patch is usually 100-10000/cm². The microneedles are attached to the skin, and the needle tip can penetrate into the stratum corneum to realize the intradermal controlled release of drugs.

The present disclosure further comprises a controlled-release method, which includes allowing the controlled release nanomaterial or the functionally active molecule nano-drug delivery system of the present disclosure to fully contact with the corresponding substrate, so as to achieve the purpose of controlled release.

The present disclosure further comprises the use of the mesoporous silica nanomaterial for controlled release or the functionally active molecule nano-drug delivery system in the manufacture of a medicine or a diagnostic reagent or diagnostic kit for controlled release of a drug. The drug for controlled release administration can be used for treating various diseases, and the diagnostic reagent or diagnostic kit can be used for diagnosing various diseases, depending on the functionally active molecules loaded in the controlled release nanomaterial or the functionally active molecule nano-drug delivery system.

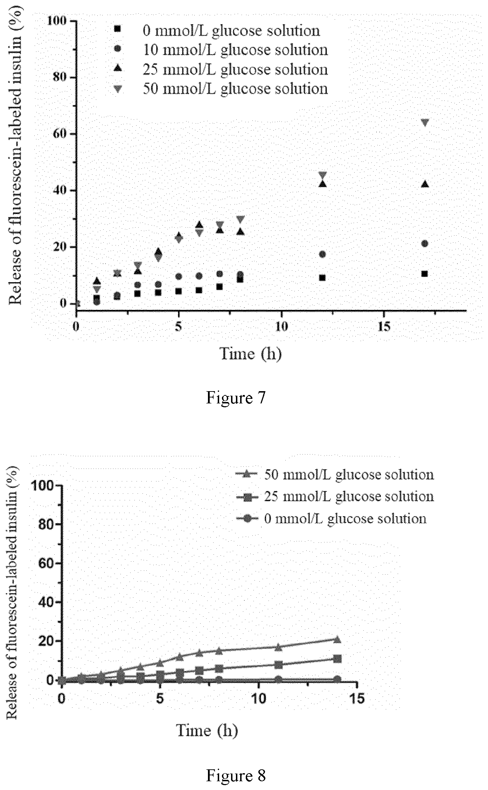

For example, when a therapeutic agent for diabetes such as insulin is loaded, the controlled release nanomaterial or functionally active molecule nano-drug delivery system of the present disclosure can be used in the manufacture of drugs for treating diabetes and its complications or controlling blood sugar levels in patients (controlled release based on blood sugar concentration range as shown in Figure 7).

The mesoporous silica-based controlled release nanomaterial or functionally active molecule nano-drug delivery system involved in the present disclosure can be applied to the living body in any suitable way and dose in any suitable form of pharmaceutical preparation. For example, when applied in vivo, it can be administered orally, locally, intravenously or intramuscularly, or in form of microneedles, microneedle patches, implants, or exogenous pumps.

In some embodiments, the controlled release nanomaterial or functionally active molecule nano-drug delivery system of the present disclosure can achieve "zero early release" of the load molecule (such as insulin) in the absence of a stimulus (substrate).

In certain embodiments, the controlled release nanomaterials or functionally active molecule nano-drug delivery systems corresponding to the same phenylboronic acid ester structure described in the present disclosure have different response concentration ranges with respect to different substrates. In practical applications, the skilled person can select an appropriate amount according to the actual concentration range of the substrate and the required content of the internal load, that is, the functionally active molecule.

In certain embodiments, the controlled release nanomaterials or functionally active molecule nano-drug delivery systems corresponding to different phenylboronic acid ester structures described in the present disclosure have different response degrees to the same substrate, and the contents of the released internal loads are also different. In practical applications, the skilled person can select the appropriate structure and dosage according to the actual concentration range of the substrate and the required content of internal load.

The present disclosure further provides use of the polyhydroxy compound described in the present disclosure and optionally the phenylboronic acid compound described in the present disclosure in the preparation of controlled release nanomaterial or functionally active molecule nano-drug delivery system.

The present disclosure will be illustrated by way of specific examples below, the purpose of which is to better understand the contents of the present disclosure. It should be understood that these examples are illustrative only and not restrictive. Reagents used in the examples, unless otherwise stated, are routinely purchased from the market. Its usage and dosage can be used according to conventional usage and dosage. Unless otherwise specified, the experimental methods, experimental conditions and detection methods and detection conditions in the examples adopt those in conventional methods and conditions in the art.

### Example 1

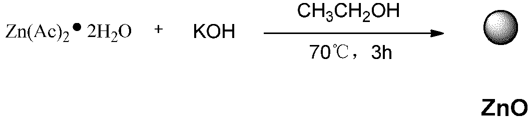

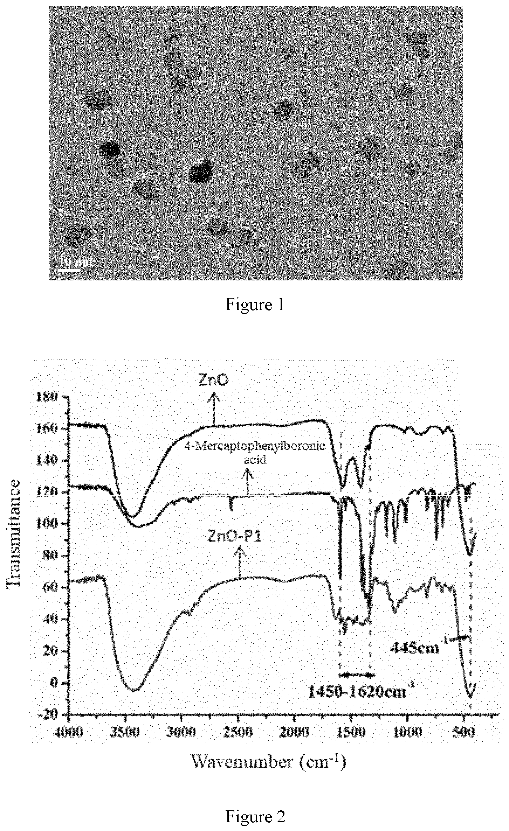

3.95 g of zinc acetate dihydrate and 100 mL of ethanol were added into a 500 mL three-neck round bottom flask, and heated to 70 °C under mechanical stirring. After the reaction solution was clear and transparent, 2.10 g KOH was weighed and dissolved in 90 mL ethanol, and added dropwise to the above system. After the addition of drop is completed within half an hour, the reaction was performed with continuously mechanical stirring (600 rpm) at 70 °C for 3 h. After 3 h, heating and mechanical stirring were stopped, and the reaction solution was taken and centrifuged at 12,000 rpm for 10 min. The supernatant was discarded, and the precipitate was washed with ethanol three times, and lyophilized to obtain 1.2 g of white solid (nano-zinc oxide ZnO). The transmission electron microscope image of the nano-zinc oxide prepared in Example 1 is shown in Figure 1. It can be seen from Figure 1 that the size of nano-zinc oxide is about 160nm.

### Example 2

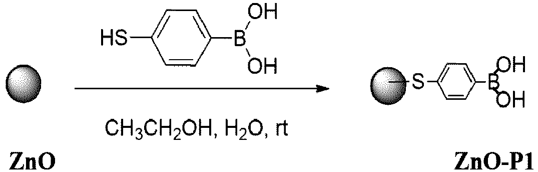

189 mg of 4-mercaptophenylboronic acid and 50 mL of ultrapure water were added into a 250 mL single-necked round bottom flask, and stirred magnetically at room temperature. 200 mg nano-zinc oxide ZnO was ultrasonic dispersed in ethanol and added dropwise to the above system. The magnetic stirring was continued at room temperature for 30 min. The reaction was stopped, and the reaction solution was taken and centrifuged at 12000 rpm for 10 min. The supernatant was discarded, and the precipitate was washed 3 times with ethanol, and lyophilized to obtain 180 mg of a yellow solid, namely nano-zinc oxide ZnO (ZnO-P1). The infrared spectra of ZnO-P1, 4-mercaptophenylboronic acid and nano-zinc oxide are shown in Figure 2. It can be seen from Figure 2 that in ZnO-P1, 4-mercaptophenylboronic acid has been modified to the surface of nano-zinc oxide.

### Example 3

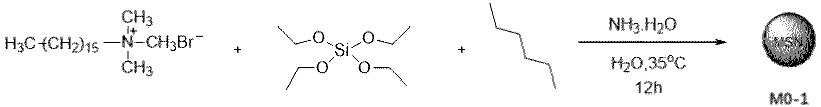

Cetyltrimethylammonium bromide (CTAB) (1.00 g, 2.7 mmol), 160 mL deionized water and ammonia water (5 mL, 70.8 mmol) were added into a 500 mL single-necked round bottom flask, and stirred magnetically at 35 °C until clear. 20 mL of n-hexane and 5 mL of tetraethyl orthosilicate were measured in a 50 mL beaker, stirred evenly, and added dropwise to the above system with a constant pressure dropping funnel within 30 min. The reaction was performed at 35 °C for 12h after the dropwise addition. After heating was stopped, the reaction liquid was filtered with suction. The filter cake was washed three times with ethanol and deionized water, and dried in an oven at 100 °C for 8 h to obtain 2.15 g of white powdery solid M0-1.

### Example 4

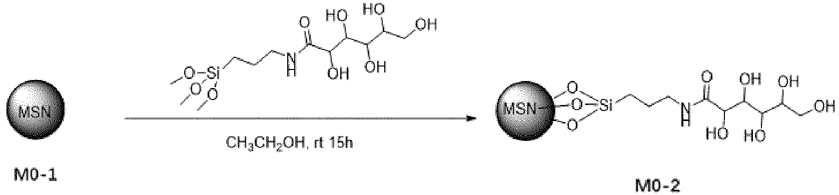

1.00 g of mesoporous silica nanoparticles M0-1 that were ground into a fine powder were added into a 500 mL single-necked round-bottom flask, followed by 100 mL of absolute ethanol, and uniformly dispersed with ultrasonication. 1 mL of N-(3-trimethoxy silylpropyl) glucamide was added and magnetically stirred at room temperature for 15 h under the protection of argon. After the reaction was stopped, the reaction liquid was filtered with suction. The filter cake was washed three times with ethanol and deionized water, and dried in an oven at 80 °C for 8 h to obtain 0.917 g of white powdery solid M0-2.

### Example 5

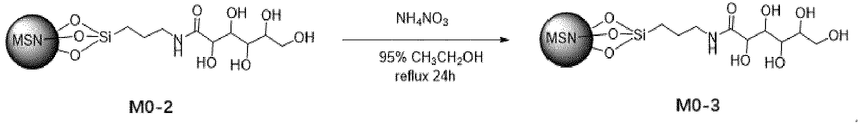

200 mg of mesoporous silica nanoparticles M0-2 that were ground into a fine powder and 47.5 mL of absolute ethanol were added into a 100 mL single-necked round bottom flask, and dispersed by ultrasonication. 500 mg of ammonium nitrate was weighed and dissolved in 2.5 mL of ultrapure water, and added into the above system for reaction under reflux for 24 h. After the reaction was stopped, the reaction liquid was filtered with suction. The filter cake was washed three times with ethanol and deionized water, and dried in an oven at 80 °C for 8 h to obtain 143 mg of white powdery solid, i.e., mesoporous silica nanoparticles functionalized by polyhydroxy compound M0-3. The transmission electron microscope image of M0-3 prepared in Example 5 is shown in Figure 3. It can be seen from Figure 3 that M0-3 has a size of about 150nm and has uniform and ordered pore channels.

### Example 6

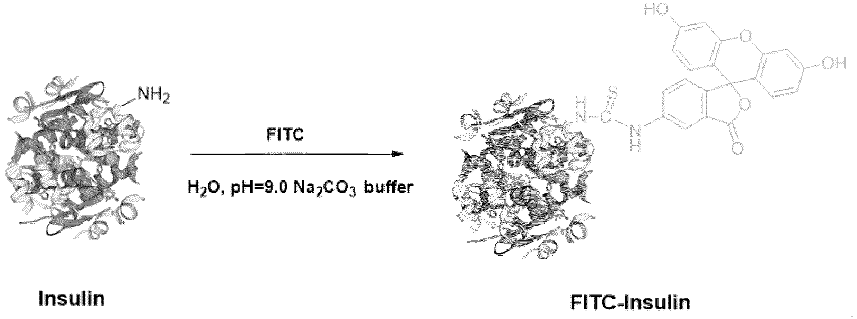

0.84 g NaHCO₃ was weighed and dissolved in 100 mL ultrapure water to make a 0.1 M NaHCO₃ solution; 1.06 g Na₂CO₃ was weighed and dissolved in 100 mL ultrapure water to make a 0.1 M Na₂CO₃ solution; 0.1 M Na₂CO₃ solution and 0.1 M NaHCO₃ solution were measured in a ratio of 1: 9 by volume respectively, and prepared into a Na₂CO₃ buffer solution with pH=9.0.

200 mg insulin was weighed and dissolved in 50 mL 0.1 M Na₂CO₃ buffer. Then fluorescein isothiocyanate (FITC) (2.5 mg, 6.4 µmol) was weighed and dissolved in 2.5 mL DMSO, and added dropwise to the above insulin solution. The reaction liquid was magnetically stirred at room temperature for 2 h protected from light. After the end of the reaction, the reaction liquid was transferred to a dialysis bag (MWCO=1000 D), dialyzed for 48 h, and the dialysate was replaced every 6 h. Finally, 188 mg of yellow solid was obtained by freeze-drying. The yellow solid was fluorescein-labeled insulin (FITC-Insulin), which was refrigerated for use.

### Example 7

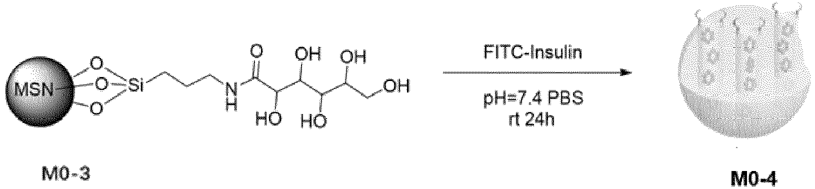

10 mg of M1-3 was weighed into a plastic centrifuge tube, and dispersed with ultrasonication after 2 mL of PBS buffer (pH 7.4) was added. 5 mg of fluorescein-labeled insulin (FITC-Insulin) was weighed, dissolved after adding 1 mL of PBS (pH 7.4) buffer solution, and added to the above system. The reaction liquid was magnetically stirred at room temperature for 24 h protected from light to obtain a mixed liquid of mesoporous silica nanoparticles M0-4 loaded with fluorescein-labeled insulin.

### Example 8

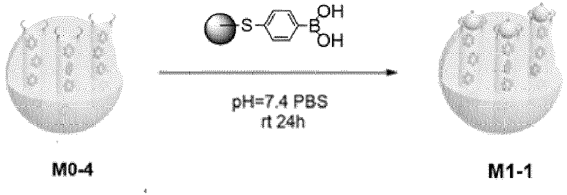

10 mg of ZnO-P1 was weighed into a plastic centrifuge tube, followed by 2 mL of PBS buffer (pH 7.4) and 200 µL of DMSO, dispersed with ultrasonication, and added to the M0-4 mixed liquid of Example 7. The reaction liquid was continuously magnetical stirred protected from light at room temperature for 24 h. Then, the reaction solution was taken and centrifuged (10000 rpm, 10 min). The supernatant was discarded, the precipitate was washed three times with PBS, and refrigerated for later use to obtain mesoporous silica nanoparticles M1-1 that were loaded with fluorescein-labeled insulin and of which the pores were blocked with nano-zinc oxide. The transmission electron microscope image of M1-1 prepared in Example 8 is shown in Figure 4. It can be seen from Figure 4 that in M1-1, the mesoporous openings on the surface of the mesoporous silica nanoparticles have been blocked by nano-zinc oxide.

### Example 9

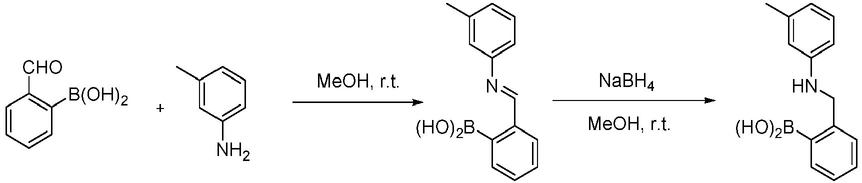

2-formylphenylboronic acid (1.000 g, 6.66 mmol) and m-toluidine (0.314 g, 6.66 mmol) were added into a 25 mL round bottom flask, followed by 10 mL of methanol, stirred at room temperature for 20 min, and cooled to 0 °C in an ice bath. Sodium borohydride (0.302 g, 8.00 mmol) was weighed and gradually added to the above reaction system in batches. The reaction was stopped after 2 hours. Saturated aqueous ammonium chloride solution was added to quench the reaction. After the pH was adjusted to neutral and methanol was removed by rotary evaporation, the reaction liquid was extracted with ethyl acetate (50 mL × 3). The organic phases were combinedand dried over anhydrous sodium sulfate. The solvent was removed by rotary evaporation. After separation by column chromatography (EA/PE=1/5(v/v)), a slightly yellow oily liquid ( 0.701 g, yield 43.7%) was obtained. ¹H NMR (400 MHz, DMSO-*d₆*): *δ* (ppm) 9.29 (s, 1H), 7. 86 (d, J=7.2 Hz, 1H), 7.43-7.38 (m, 4H), 7.39-7.30 (m, 1H), 7.16 (t, *J*=8.0 Hz, 1H), 6.72 (d, J=7.6 Hz, 1H), 4.51 (s, 2H), 2.30 (s, 3H).

### Example 10

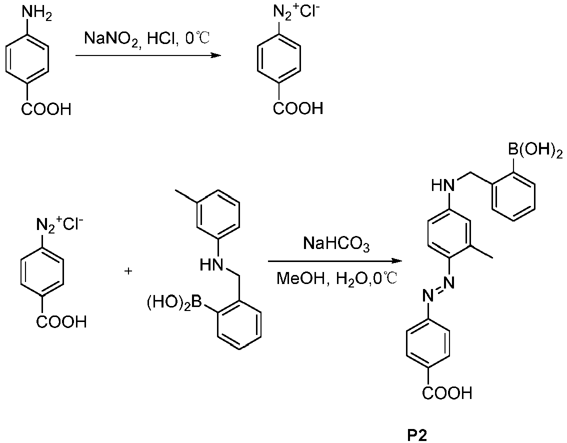

4-aminobenzoic acid (0.905 g, 6.60 mmol) was added into a 50 mL round-bottomed flask, followed by 10 mL hydrochloric acid (5M), and stirred magnetically under ice bath conditions. The solution turned white and turbid. 4-aminobenzoic acid (0.455 g, 6.60 mmol) was weighed and dissolved in 4 mL ultrapure water and added dropwise to the above system with a constant pressure dropping funnel. The reaction system was kept stirring magnetically under ice bath conditions until the solution gradually became clear. The product in Example 9 (1.061 g, 4.40 mmol) was put in a 250 mL round-bottomed flask, and dissolved by adding 10 mL of methanol, and 10 mL of HCl solution (1M). The solution was added dropwise to the clear solution containing diazonium salt until the solution gradually turned into a dark red turbid liquid. After the dropwise addition, 40 mL of NaHCO₃ saturated solution was added, and the reaction was stopped after 1 h. After suction filtration, the filter cake was washed with a large amount of ultrapure water. After separation by column chromatography (CH₂Cl₂/MeOH=50/1 (v/v)), dark red solid P2 (0.864 g, yield 50.5%) was obtained. ¹H NMR (400 MHz, DMSO-d6): *δ* (ppm) 8.13-8.04 (m, 2H), 7. 92 (d, *J*=8.0 Hz, 1H), 7.82 (d, *J*=8.0 Hz, 2H), 7.72-7.57 (m, 2H), 7.48 (d, *J*=4.0 Hz, 1H), 7.35-7.24 (m, 2H), 6.68 (s, 1H), 4.65(s, 2H), 2.74(s, 1H), 2.56(s, 2H).

### Example 11

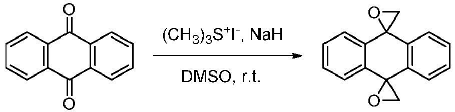

Anthraquinone (2.08 g, 10 mmol) and sodium hydride (1.2 g, 30 mmol, 60%) were added to a 250 mL round-bottom flask, followed by 60 mL of dimethyl sulfoxide, and magnetic stirred at room temperature protected from light. Trimethylsulfonium iodide (6.12 g, 30 mmol) was weighed, dissolved in 40 mL of dimethyl sulfoxide, and gradually dropped into the above light-protected reaction system in a constant pressure drop funnel. After the addition of drop, the reaction was stopped after reacting for 2 h at room temperature protected from light. The reaction solution was poured into 200 mL of ice water, and a large amount of yellow flocs was precipitated. After standing for half an hour, the filter cake was sucked with a large amount of ultrapure water. After drying in infrared oven, it was directly used for the next reaction.

### Example 12

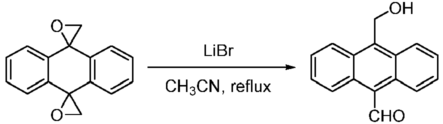

The product in Example 11 and lithium bromide (4.00 g, 46.1 mmol) were added into a 250 mL round bottom flask, followed by 150 mL of acetonitrile, and heated to reflux. After 16 h, the reaction was stopped, and the solution was yellow and transparent. The hot solution was placed in a -40 °C refrigerator for recrystallization. After 3 h, a large amount of yellow needle-like solid precipitated out of the solution. After suction filtration, the filter cake was washed with a large amount of ultrapure water, and dried in an infrared oven to obtain a yellow crystalline solid (1.417 g) and the total yield of the two steps was 60%. ¹H NMR (400 MHz, DMSO-*d₆*): *δ* (ppm) 11.46 (s, 1H), 8.95 (d, *J*=8.8 Hz, 2H), 8.61 (d, *J*=8.4 Hz, 2H), 7.75-7.65 (m, 4H), 5.58 (t, *J*=5.2 Hz, 1H), 5.47 (d, *J*=5.2 Hz, 2H).

### Example 13

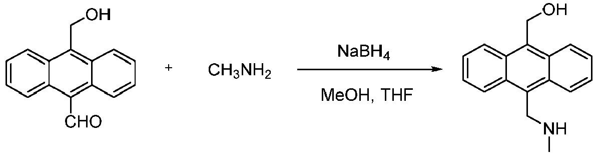

The product in Example 12 (474 mg, 2.0 mmol) was added into a 100 mL round-bottomed flask, followed by 25 mL of anhydrous methanol and 15 mL of tetrahydrofuran, stirred at room temperature, and the solution became a yellow suspension. 8 mL of aqueous methylamine solution (25%-30%, wt) was added. The solution gradually turned into a yellow clear liquid. After 16 h, sodium borohydride (378 mg, 10.0 mmol) was added in batchs to the above reaction system. After 1 h, 30 mL of saturated aqueous ammonium chloride solution was added to quench the reaction, and the solvent was removed by rotary evaporation. The reaction liquid was extracted with ethyl acetate (50 mL × 5), and the organic phases were combined and dried over anhydrous sodium sulfate. The organic solvent was removed by rotary evaporation. After separation by column chromatography (CH₂Cl₂/MeOH=50/1 (v/v)), a light yellow powdery solid (0.319 g, yield 63.5%) was obtained. ¹H NMR (400 MHz, DMSO-*d₆*): *δ* (ppm) 8.50-8.43 (m, 4H), 7.57-7.54 (m, 4H), 5.44 (s, 2H), 4.57 (s, 2H), 3.27 (s, 3H).

### Example 14

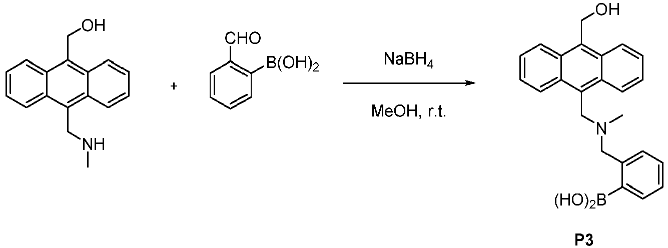

The product in Example 13 (122 mg, 0.485 mmol) and 2-formylphenylboronic acid (73 mg, 0.485 mmol) were added into a 50 mL two-necked flask, followed by 8 mL of methanol, and stirred magnetically at room temperature in a closed state. The solution was pale yellow and clear. After 45 h, sodium borohydride (92 mg, 2.425 mmol) was added in batches under ice bath. Saturated aqueous ammonium chloride solution was added for 1 h to quench the reaction. The solvent was removed by rotary evaporation. The reaction liquid was extracted with dichloromethane (50 mL × 5). The organic phases were combined and dried over anhydrous sodium sulfate. The organic solvent was removed by rotary evaporation. After separation by column chromatography (CH₂Cl₂/MeOH=50/1 (v/v)), a pale yellow solid P3 (90 mg, yield 48.4%) was obtained. ¹H NMR (400 MHz, CD₃OD): *δ* (ppm) 8.55 (d, *J*=8.0 Hz, 2H), 8.22 (d, *J*= 8.0 Hz, 2H), 7.72 (d, *J*= 8.0 Hz, 1H), 7.62-7.53 (m, 4H), 7.41-7.36 (m, 2H), 7.30 (t, *J*= 8.0 Hz, 1H), 5.61(s, 2H), 5.06 (s, 2H), 4.38 (s, 2H), 2.41 (s, 3H).

### Example 15

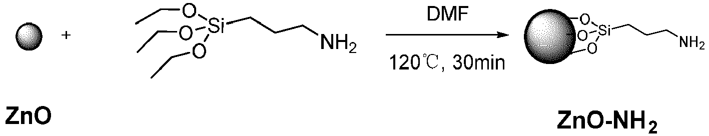

3.95 g of zinc acetate dihydrate and 100 mL of ethanol were added into a 500 mL three-neck round bottom flask, and heated to 70 °C under mechanical stirring. After the reaction solution was clear and transparent, 2.10 g KOH was weighed and dissolved in 90 mL ethanol, and added dropwise to the above system. After the drop was completed within half an hour, mechanical stiring (600 rpm) was continued and the reaction was kept at 70 °C for 3 h. After 3 h, 5 mL of 3-aminopropyltriethoxysilane (APTES) was added dropwise to the above system, and mechanical stirring was continued for 1 h. After 1 h, heating and mechanically stirring were stopped, and the reaction solution was taken and centrifuged at 12,000 rpm for 10 min. The supernatant was discarded. The precipitate was washed with ethanol three times, and lyophilized to obtain 1.2 g of white solid ZnO-NH₂.

### Example 16

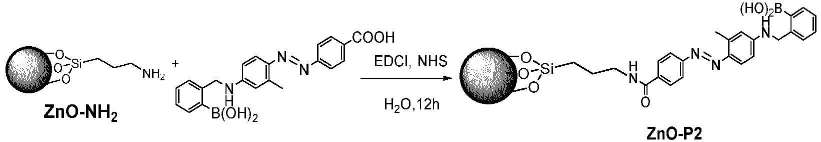

P2 (10 mg, 0.0257 mmol), 1-ethyl-(3-dimethylaminopropyl)carbodiimide hydrochloride EDCl (7.4 mg, 0.0385 mmol), N-hydroxysuccinimide NHS (4.4 mg, 0.0385 mmol) and 30 mL of ultrapure water were added to a 100 mL single-necked round bottom flask, adjusted to pH 7.0 with dilute NaOH, and magnetically stirred at room temperature for 4 h. 300 mg ZnO-NH² was dispersed with ultrasonication in ultrapure water and added to the above system. Magnetic stirring was continued at room temperature for 4 h. After 4 h, the reaction was stopped, the reaction solution was centrifuged at 12000 rpm for 10 min. The supernatant was discarded. The precipitate was washed 3 times with ethanol, and lyophilized to obtain 280 mg red solid ZnO-P2 (nano-zinc oxide functionalized by phenylboronic acid).

### Example 17

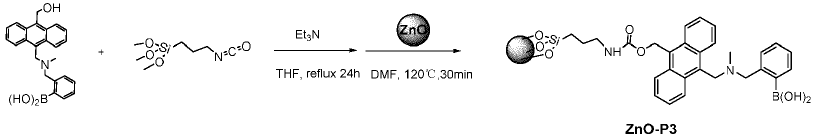

In a 50 mL two-necked round bottom flask, P3 (12.2 mg, 0.0317 mmol), (3-isocyanopropyl)trimethoxysilane (15.7 mg, 0.0634 mmol), 10 mL of anhydrous tetrahydrofuran and 2-3 drops of tris ethylamine were added, and refluxed for 24 h under the protection of argon. After 24 h, the solvent was removed by rotary evaporation to obtain a yellow oil, which was dissolved in 10 mL of anhydrous DMF and set aside. 300 mg ZnO was weighed and ground into a fine powder, and ultrasonically dispersed in 40 mL of anhydrous DMF. The DMF solution of the above oil was added, and stirred magnetically at 120 °C for 30 min. After 30 min, heating and stirring were stopped. After cooled to room temperature, the reaction solution was centrifuged at 12000 rpm for 10 min. The supernatant was discarded. The precipitate was washed with ethanol three times, and lyophilized to obtain 274 mg off-white solid ZnO-P3 (nano-zinc oxide functionalized by phenylboronic acid).

### Example 18

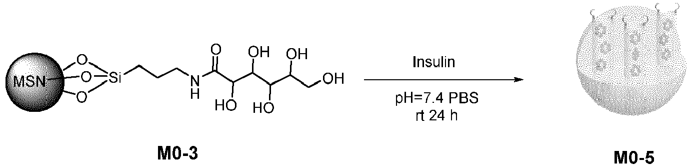

10 mg of M0-3 was weighed into a plastic centrifuge tube. 2 mL of PBS buffer (pH 7.4) was added and ultrasonically dispersed. 5 mg of insulin was weighed and dissolved after adding 1 mL of PBS buffer (pH 7.4), and added to the above system. After the system was stirred magnetically for 24 h at room temperature protected from light, a mixed liquid of mesoporous silica nanoparticles M0-5 loaded with insulin- (not labeled with fluorescein) was obtained.

### Example 19

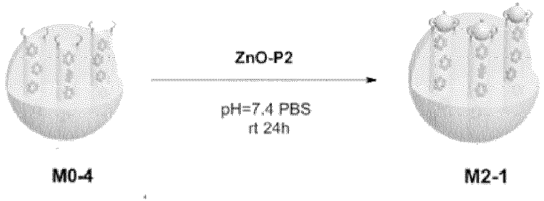

10 mg of ZnO-P2 was weight into a plastic centrifuge tube, dispersed with ultrasonication after 2 mL of PBS buffer (pH 7.4) and 200 µL of DMSO were added, added to the mixed liquid M0-4 in Example 7, and continuously magnetic stirred at room temperature for 24 h protected from light. Then, the reaction solution was taken and centrifuged (10000 rpm, 10 min). The supernatant was discarded. The precipitate was washed three times with PBS, and refrigerated for later use, to obtain mesoporous silica nanoparticles M2-1 that were loaded with fluorescein-labeled insulin and of which the pores were blocked with nano-zinc oxide.

### Example 20

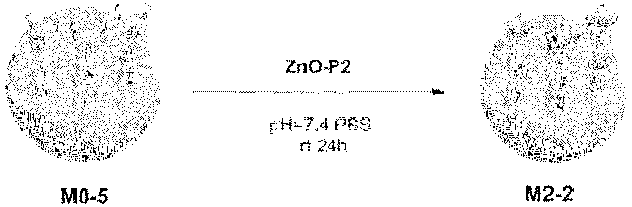

10 mg of ZnO-P2 was weighed into a plastic centrifuge tube, dispersed by ultrasonication after 2 mL of PBS buffer (pH 7.4) and 200 µL of DMSO were added, added to the M0-5 mixed liquid in Example 18, and continuously magnetic stirred at room temperature for 24 h protected from light. Then, the reaction solution was taken and centrifuged (10000 rpm, 10 min). The supernatant was discarded. The precipitate was washed three times with PBS, and refrigerated for later use to obtain mesoporous silica nanoparticles M2-2 that are loaded with insulin (not labeled with fluorescein) and of which the pores were blocked with nano-zinc oxide.

### Example 21

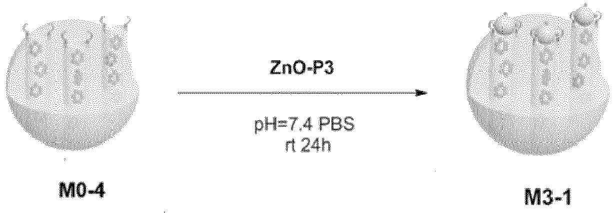

10 mg of ZnO-P3 was weighed into a plastic centrifuge tube, dispersed by ultrasonication after 2 mL of PBS buffer (pH 7.4) and 200 µL of DMSO were added, added to the above M0-4 mixed liquid, and continuously magnetic stirred at room temperature for 24 h protected from light. Then, the reaction solution was taken and centrifuged (10000 rpm, 10 min). The supernatant was discarded. The precipitate was washed three times with PBS, and refrigerated for later use to obtain mesoporous silica nanoparticles M3-1 that were loaded with fluorescein-labeled insulin and of which the pores are blocked with nano-zinc oxide.

### Example 22

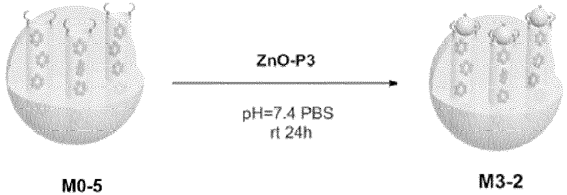

10 mg of ZnO-P3 was weighed into a plastic centrifuge tube, dispersed by ultrasonication after 2 mL of PBS buffer (pH 7.4) and 200 µL of DMSO were added, and added to the above M0-5 mixed liquid, and continuously magnetic stirred at room temperature for 24 h protected from light. Then, the reaction solution was taken and centrifuged (10000 rpm, 10 min). The supernatant was discarded, the precipitate was washed three times with PBS, and refrigerated for later use to obtain mesoporous silica nanoparticles M3-2 that were loaded with insulin (not labeled with fluorescein) and of which the pores are blocked with nano-zinc oxide.

### Example 23

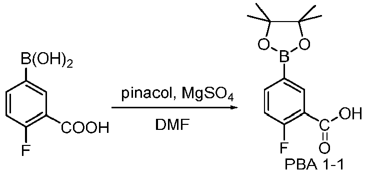

3-carboxy-4 fluorophenylboronic acid (5.0 g), pinacol (4.8 g) and DMF 20 mL were added into a 100 mL round bottom flask, stirred magnetically at room temperature for 15 min, followed by anhydrous magnesium sulfate (4.9 g) was added, and reacted overnight. The reaction was stopped, extracted with EA, spin-dried, and the resulting solid was separated by column chromatography (DCM) to obtain 4.5 g of PBA1-1 as a white solid. ¹H NMR (400 MHz, DMSO) *δ* 7.87 (t, *J=* 7.4 Hz, 1H), 7.56 (d, *J=* 7.6 Hz, 1H), 7.42 (d, *J* = 11.0 Hz, 1H), 1.31 (s, 12H).

### Example 24

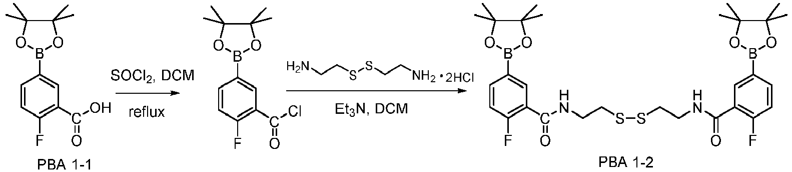

PBA1-1 (1.0 g, 3.759 mmol), 0.41 mL (5.639 mmol) of dichlorosulfoxide and 5 mL of anhydrous DCM were added to a 50 mL round bottom flask, protected by argon, and stirred at 60°C for 4 h at reflux. The reaction was stopped, and the solvent was spin-dried, and 5 mL of anhydrous DCM was added for later use. In another 50 mL double-necked round bottom flask, 380 mg (1.6915 mmol) of cystamine dihydrochloride, 1.56 mL of triethylamine and 10 mL of anhydrous DCM were added, under argon protection, and magnetically stirred at room temperature for 15 min. Under the condition of ice bath, the DCM solution containing acid chloride was slowly added to the above system, and the reaction was continued for 6h. After the reaction was stop, the solvent was spin dried, extracted with EA, washed with saturated brine three times, and the organic solvent was spin dried to obtain 650 mg of PBA1-2 as a yellow solid with a yield of 54%. ¹H NMR (400 MHz, DMSO) *δ* 8.54 (s, 2H), 7.63 (t, *J=* 7.3 Hz, 2H), 7.53 (d, *J=* 7.6 Hz, 2H), 7.40 (d, *J* = 10.6 Hz, 2H), 3.56 (dd, *J* = 12.7, 6.4 Hz, 4H), 2.93 (t, *J* = 6.8 Hz, 4H), 1.31 (s, 24H).

### Example 25

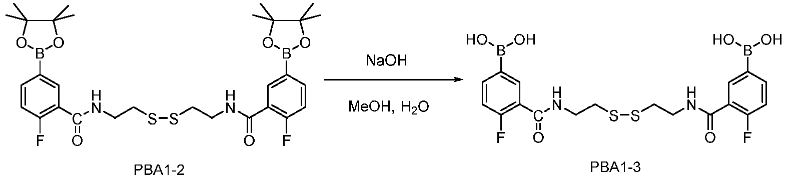

PBA1-2 (1.27 g), NaOH (5.08 g), 63.5 mL of ultrapure water and 32.0 mL of methanol were added to a 25 mL round bottom flask, and stirred magnetically at room temperature for 48 h. After t he reaction was stopped, the methanol solvent was spin-dried. The remaining solution was acidified with 0.1 M HCl, a large amount of white solid was precipitated, and filtered by suction. The obtained solid was dried by infrared column, and separated by column chromatography (DCM/MeOH=20:1) to obtain a white solid PBA1-3 (560 mg, yield 86 %).¹H NMR (400 MHz, DMSO) *δ* 8.55 (s, 2H), 7.67 (dd, *J=* 10.0, 1.5 Hz, 2H), 7.60 - 7.47 (m, 4H), 3.55 (dd, *J=* 12.7, 6.4 Hz, 4H), 2.92 (t, *J=* 6.8 Hz, 4H).

### Example 26

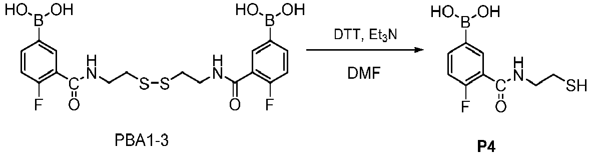

PBA1-3 (100 mg), dithiothreitol (37 mg), 0.5 mL of triethylamine and 5 mL of anhydrous DMF were added into a 25 mL round bottom flask, protected under argon, stirred magnetically for 3 h at room temperature Column chromatography (DCM/MeOH=20:1) was performed to obtain a white solid P4 (10 mg, yield 20%).¹H NMR (400 MHz, DMSO) *δ* 8.54 (s, 1H), 7.67 (dd, *J=* 10.0, 1.5 Hz, 1H), 7.57 (t, *J=* 7.9 Hz, 1H), 7.51 (dd, *J=* 8.3, 1.6 Hz, 1H), 3.55 (dd, *J=* 12.7, 6.4 Hz, 2H), 2.92 (t, *J=* 6.8 Hz, 2H), 2.50 (s, 2H).

### Example 27

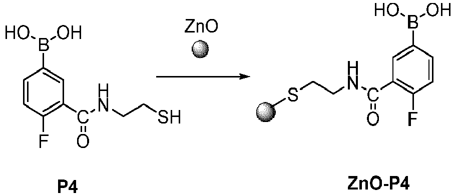

263 mg P4 and 50 mL DMF were added into a 250 mL single-necked round bottom flask, and stirred magnetically at room temperature. 200 mg nano-zinc oxide ZnO in ethanol was ultrasonically dispersed and added dropwise to the above system. Magnetic stirring was continued at room temperature for 30 min. After the reaction was stopped, the reaction solution was centrifuged at 12000 rpm for 10 min. The supernatant was discarded. The precipitate was washed 3 times with ethanol and lyophilized to obtain 170 mg of a yellow solid ZnO-P4 (nano-zinc oxide functionalized by phenylboronic acid).

### Example 28

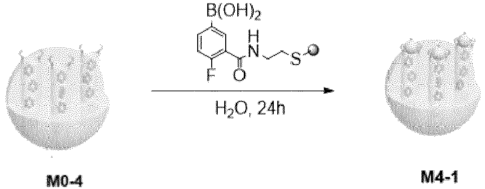

10 mg of ZnO-P4 was weighed into a plastic centrifuge tube, dispersed by ultrasonication after 2 mL of PBS buffer (pH 7.4) and 200 µL of DMSO were added, and added to the above M0-4 mixed liquid. Magnetic stirring was continued at room temperature for 24 h protected from light. Then, the reaction solution was taken and centrifuged (10000 rpm, 10 min). The supernatant was discarded. The precipitate was washed three times with PBS, and refrigerated for later use to obtain mesoporous silica nanoparticles M4-1 that were loaded with fluorescein-labeled insulin and of which the pores were blocked with nano-zinc oxide.

### Example 29

The loaded M1-1 was taken into a 7 mL plastic centrifuge tube. PBS buffer (pH 7.4) was added to wash the insulin adsorbed on the surface. After the completion of washing, 4 mL of PBS buffer (pH 7.4) was added and stirred magnetically at 37 °C. The fluorescence intensity of the supernatant was measured. After the fluorescence intensity of the supernatant remained unchanged, glucose with certain concentrations (0 mM, 25 mM, 50 mM, 100 mM, 300 mM, 500 mM) was added. After a certain time interval, the reaction liquid was centrifuged. 3 mL of the supernatant was taken and tested for its fluorescence intensity (Ex=488 nm, Em=520 nm), and the supernatant was transferred back to the original test system after the test was completed. The test results of Example 29 are shown in Figure 5. It can be known from Figure 5 that the release rate of FITC-insulin in M1-1 increases with the increase of glucose concentration.

### Example 30

The loaded M2-1 was taken into a 7 mL plastic centrifuge tube. PBS buffer (pH 7.4) was added to wash the insulin adsorbed on the surface. After the completion of washing, 4 mL of PBS buffer (pH 7.4) was added and stirred magnetically at 37 °C. The fluorescence intensity of the supernatant was measured. After the fluorescence intensity of the supernatant remained unchanged, glucose with certain concentrations (0 mM, 25 mM, 50 mM, 100 mM) was added. After a certain time interval, the reaction liquid was centrifuged. 3 mL of the supernatant was taken and tested for its fluorescence intensity (Ex=488 nm, Em=520 nm), and the supernatant was transferred back to the original test system after the test was completed. The test results of Example 30 are shown in Figure 6. It can be known from Figure 6 that the release rate of FITC-insulin in M2-1 increases with the increase of glucose concentration, and the concentration of M2-1 in response to glucose is closer to the blood sugar range of diabetic patients

### Example 31

The loaded M3-1 was taken into a 7 mL plastic centrifuge tube. PBS buffer (pH 7.4) was added to wash the insulin adsorbed on the surface. After the completion of washing, 4 mL of PBS buffer (pH 7.4) was added and stirred magnetically at 37 °C., The fluorescence intensity of the supernatant was measured. After the fluorescence intensity of the supernatant remained unchanged, glucose with certain concentrations (0 mM, 10 mM, 15 mM, 50 mM) was added. After a certain time interval, the reaction liquid was centrifuged. 3 mL of the supernatant was taken and tested for its fluorescence intensity (Ex=488 nm, Em=520 nm), and the supernatant was transferred back to the original test system after the test was completed. The test results of Example 31 are shown in Figure 7. It can be known from Figure 7 that the release rate of FITC-insulin in M3-1 increases with the increase of glucose concentration, and the concentration of M3-1 in response to glucose is closer to the blood sugar range of diabetic patients

### Example 32

The loaded M4-1 was taken into a 7 mL plastic centrifuge tube. PBS buffer (pH 7.4) was added to wash the insulin adsorbed on the surface. After the completion of washing, 4 mL of PBS buffer (pH 7.4) was added and stirred magnetically at 37 °C. The fluorescence intensity of the supernatant was measured. After the fluorescence intensity of the supernatant remained unchanged, glucose with certain concentrations (0 mM, 25 mM, 50 mM) was added. After a certain time interval, the reaction liquid was centrifuged. 3 mL of the supernatant was taken and tested for its fluorescence intensity (Ex=488 nm, Em=520 nm), and the supernatant was transferred back to the original test system after the test was completed. The test results of Example 32 are shown in Figure 8. It can be known from Figure 8 that the release rate of FITC-insulin in M4-1 increases with the increase of glucose concentration.

### Example 33

The well-growing Chang Liver cells were inoculated into 96-well plates, and the cell density observed under a microscope was about 50%. The cells were cultured in a cell incubator (37°C, 5% CO₂) for 8-12 hours to allow them to grow on the wall.

The next day, 1.0 mg of M0-3, M0-4 and M1-1 were weighed respectively. 2 mL of DMEM medium was added and ultrasonically dispersed to make a 500 µg/mL stock solution. 0.8 mL of the stock solution was pipetted into 1.2 mL of DMEM medium to prepare a 200 µg/mL solution; 1.0 mL of the above solution was pipetted into 1.0 mL DMEM medium to prepare a 100 µg/mL solution, and was gradually diluted by analogy to obtain 5 suspensions with different concentrations of 200 µg/mL, 100 µg/mL, 50 µg/mL, 25 µg/mL and 12.5 µg/mL. The culture medium in the 96-well plate was discarded, and 200 µL of the above 5 suspensions with different concentrations of nanoparticles were added to each well, and four replicate wells were repeated, and cultured in a cell culture incubator (37°C, 5% CO₂) for 24 h.

After 24 h, 20 µL of MTT solution (5 mg/mL) was added to each well, and continuously cultured in a cell incubator (37°C, 5% CO₂) for 4 h. Succinate dehydrogenase in the mitochondria of living cells can reduce MTT to blue-purple crystalline formazan and deposit it in the cell.

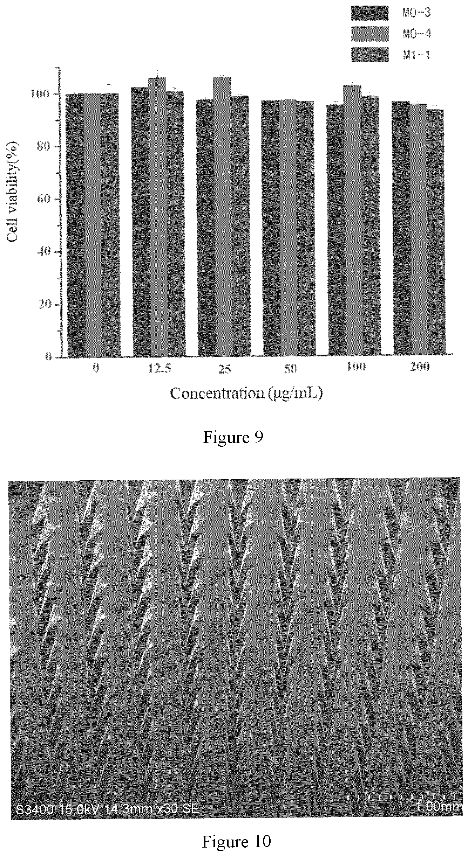

After 4 hours, the medium in the 96-well plate was discarded. 200 µL DMSO was added to each well to dissolve formazan. The absorbance (492 nm, 570 nm, 630 nm) was measured in a microplate reader and the cell survival rate was calculated. The results are shown in Figure 9.

### Example 34

Male wistar rats of 4-6 weeks were selected and fed for two weeks. streptozotocin (STZ) was administered once in a dose of 180 mg/kg (administered according to the specific body weight of the rat). The STZ was weighed according to the weight of the rat, put in a sterilized bottle, then wrapped with tinfoil. The sodium citrate buffer solution and the STZ bottle were brought into the animal room under ice bath conditions. A sodium citrate buffer solution with a concentration of 1% STZ (1 g/100ml) was prepared, and injected into the tail vein within 30 minutes to prevent inactivation. After injection of STZ, adequate drinking water and feed were given every day. Fasting blood sugar was measured on the 1st day, 7th day, and 10th day. If it was greater than 16.65 mmol/L, it was a stable diabetes model.

### Example 35

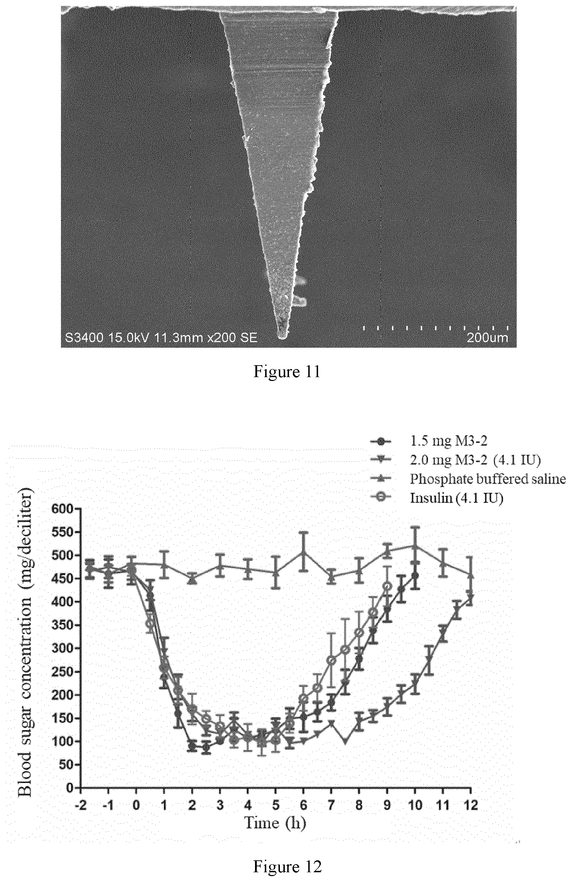

1.5 mg of M3-2 was added into 0.5 mL of ultrapure water, ultrasonically dispersed evenly. 0.25 g of hyaluronic acid (HA) was added, and stirred at room temperature until a homogeneous system was obtained to obtain an HA aqueous solution of M3-2, which was used as a pouring material of microneedle (Microneedles, MNs). 1 g of HA was added to 1 mL of ultrapure water and stirred at room temperature until clear and transparent to obtain a blank solution of HA, which was used as the base material of MNs. 50 µL HA aqueous solution of M3-2 was taken and added to the surface of the mold. The mold was placed in a 50 ml Corning tube, and centrifuged by a centrifuge (TDZ5-WS, Shanghai Xiangyi Centrifuge Instrument Co., Ltd., China) at 4390×g for 5 min; a pipette gun was used to absorb the solution on the surface of the mold, and centrifuged at 4390×g for 5 min; the mold was taken out, and dried in a dryer for 24 hours to obtain the microneedle patch (SGRM microneedle patch, SGRM patch) of the present disclosure. 1 mL of HA blank solution (1 g HA/ 1 mL H2O) was taken and put on the surface of the mold and centrifuged at 4390×g for 5 min; the mold was removed and dried in a desiccator for 24 h to obtain a blank microneedle patch (Blank patch). The photomicrographs of the microneedle patch of the present disclosure are shown in Figure 10 and Figure 11.

### Example 36

C57bl/6 mice (male, 4-6 weeks) were selected and fed adaptively for two weeks at room temperature of 24°C ± 1°C, humidity of 55% for 12 h with bright cycle (8:00-20:00) with adequate water and feed. Fasting for more than 12 hours overnight before modeling without water fasting was performed. STZ was administered once in a dose of 180 mg/kg. STZ was weighed according to the weight of the mouse, put in a sterilized bottle, then wrapped with tinfoil. The sodium citrate buffer solution and the STZ bottle were brought into the animal room under ice bath. A sodium citrate buffer solution with a concentration of 1% STZ was prepared and injected intraperitoneally within 1 minute to prevent inactivation. After the injection of STZ, fasting was continued for 2h. Afterwards, adequate drinking water and feed were given daily. Fasting blood glucose was measured on the 3rd, 5th, 7th, 10th, and 21st days, and it was a stable diabetes model if it was greater than 16.65 mmol/L.

### Example 37

Diabetic rats were taken and divided into 4 groups (4 rats in each group). The group of diabetic rats were subcutaneously injected (1) 1.5 mg M3-2, (2) 2.0 mg M3-2 (4.1 IU), (3) 1.0 mL PBS buffer solution and (4) insulin (4.1 IU). Blood sugar in rats was monitored for a period of time after injection. The experimental results are shown in Figure 12. Under the same dose, the duration of blood glucose of the rats injected with the insulin nano-drug delivery system is about 8 hours at 100-200 mg/dL, whereas the duration of blood glucose in rats injected with pure insulin is only 4.5 hours at 100-200 mg/dL, indicating that the insulin nano-drug delivery system of the present disclosure can effectively release or not release insulin according to the blood glucose of rats, thereby increasing the valid duration of insulin and preventing it from being rapidly metabolized in the body. Moreover, with the increase of the dose of the nano-drug delivery system, no obvious symptoms of hypoglycemia occurred, indicating the high reliability and stability of the nano-drug delivery system.

### Example 38

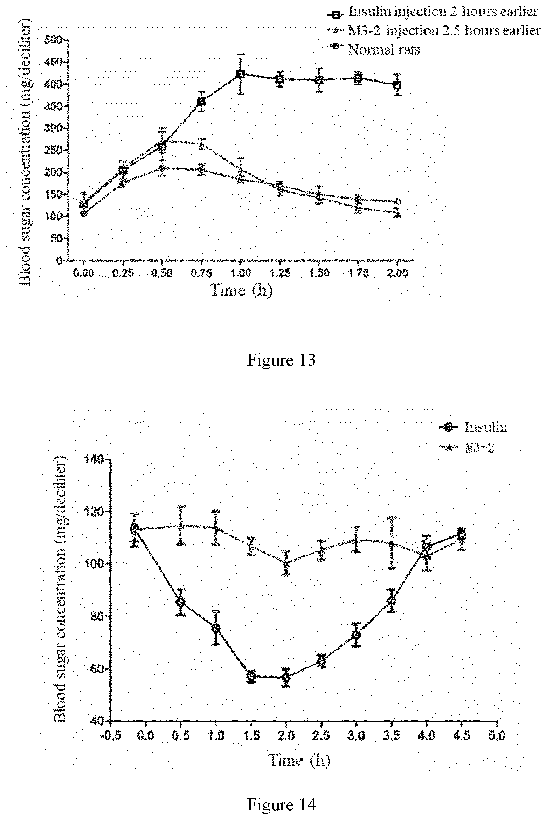

2 groups of diabetic rats and 1 group of normal rats (4 rats in each group) were taken. Diabetic rat groups were subcutaneously injected with (1) 2.0 mg M3-2 (4.1 IU) and (2) insulin (4.1 IU) in the neck respectively. After 2.5h of injection, g lucose (1.5g/kg) was intraperitoneally injected into rats in the diabetes group and the normal group, and the blood sugar was detected. The experimental results are shown in Figure 13. Compared with the normal group, the experimental group injected with the nano-drug delivery system of the present disclosure can simulate the function of normal islets, release insulin when blood sugar rises, and then regulate the blood sugar of rats, while in the experimental group injected with insulin alone, after glucose injection, the blood sugar quickly returns to a higher blood sugar level. The above results show that the insulin nano-drug delivery system of the present disclosure has obvious effect on regulating blood sugar.

### Example 39

The normal rats were divided into two groups (4 rats in each group), and (1) 2.0 mg M3-2 (4.1 IU) and (2) insulin (4.1 IU) were injected subcutaneously in the neck respectively, and the blood sugar was detected. The experimental results are shown in Figure 14. Experiments show that under the condition of normoglycemia, the insulin nano-drug delivery system of the present disclosure does not release insulin, and there is no risk of hypoglycemia, indicating the "intelligence" and safety of the nano-drug delivery system.

### Example 40

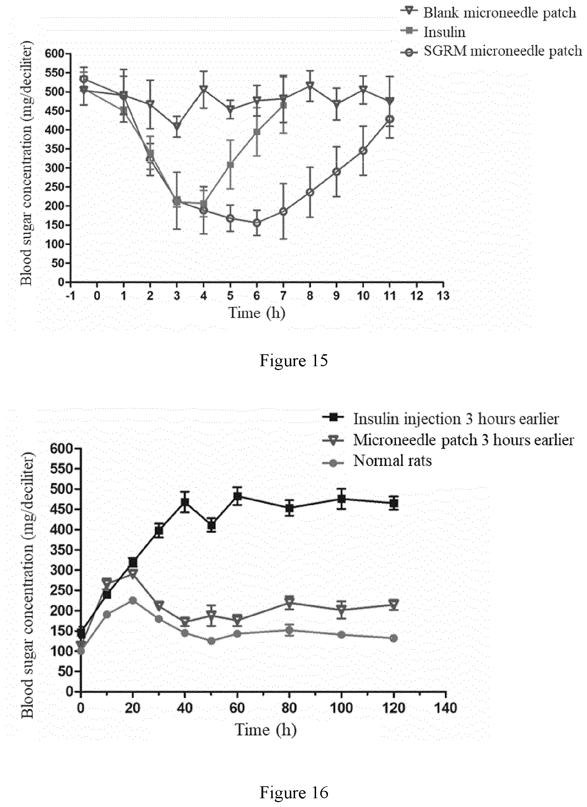

Diabetic mice were taken with their backs shaved, and divided into 3 groups (4 mice in each group). The 3 groups were subjected to (1) blank patch on the back, (2) subcutaneous injection of insulin (12.0 IU/kg) in the neck, and (3) SGRM microneedle patch (12.0 IU/kg) on the back. The blood glucose of the mice was monitored for a period of time after the injection or the patch. The experimental results are shown in Figure 15. Experiments show that the SGRM microneedle patch of the present disclosure can effectively release or not release insulin according to the blood sugar of mice, thereby increasing the effective duration of insulin and preventing it from being rapidly metabolized in the body.

### Example 41

2 groups of diabetic mice and 1 group of normal mice (4 mice in each group) were taken. Diabetic mice were shaved on the back, and received (1) subcutaneous injection of insulin (12.0 IU/kg) in the neck, and (2) SGRM microneedle patch (12.0 IU/kg) on the back. After 2.5h of injection or patch, glucose (1.5 g/kg) was intraperitoneally injected into mice in the diabetes group and the normal group, and the blood sugar was detected. The experimental results are shown in Figure 16. Experiments have shown that compared with the normal group, the experimental group administered with the SGRM microneedle patch of the present disclosure can simulate the function of normal islets, release insulin when blood sugar rises, and then regulate the blood sugar of mice. However, in the experimental group injected with insulin alone, the blood sugar quickly rises to a higher blood sugar level after glucose injection, indicating that the SGRM microneedle patch of the present disclosure has an obvious effect of regulating blood sugar.

### Example 42

Normal mice were taken with their backs shaved, and divided into 2 groups (4 rats in each group). The 2 groups were subjected to (1) b SGRM microneedle patch (12.0 IU/kg) on the back, and (2) subcutaneous injection of insulin (9.5 IU/kg) in the neck, and blood sugar was detected. The experimental results are shown in Figure 17. Experiments show that under the condition of normal blood sugar, the SGRM microneedle patch of the present disclosure does not release insulin, and there is no risk of hypoglycemia, indicating the "intelligence" and safety of the SGRM microneedle patch.

Although the present disclosure has been described in terms of specific examples, it should be understood that the scope of the present disclosure is not limited to the above specific embodiments. Without departing from the spirit and scope of the present disclosure, those skilled in the art can make various modifications and changes to the present disclosure, and these modifications and changes are within the scope of the present disclosure.

## Claims

1. A mesoporous silica nanomaterial for controlled release, wherein the mesoporous silica nanomaterial for controlled release comprises mesoporous silica nanoparticles functionalized by a polyhydroxy compound and a nanoparticle pore blocking agent functionalized by a phenylboronic acid compound.

2. The mesoporous silica nanomaterial for controlled release according to claim 1, wherein:
(1) the polyhydroxy compound has a structure as shown by R₁-R₂ , wherein R₁ is amino or carboxyl, R₂ is linear or branched C₂₋₁₂ alkyl, C₂₋₁₂ alkenyl or C₂₋₁₂ alkynyl, and the alkyl, the alkenyl and the alkynyl are substituted by at least 2 hydroxyl groups, and at least two adjacent C atoms are respectively substituted by one hydroxyl group; preferably, the polyhydroxy compound is a monosaccharide and its derivatives retaining at least two adjacent hydroxyl groups, a polysaccharide and its derivatives retaining at least two adjacent hydroxyl groups, a synthetic block copolymer comprising 2 or more hydroxyl groups and at least two of which are adjacent; more preferably, the polyhydroxy compound is selected from an open-chain glucose and its derivatives, such as gluconic acid; and/or
(2) the phenylboronic acid compound is selected from a structure shown in the following formula (A): 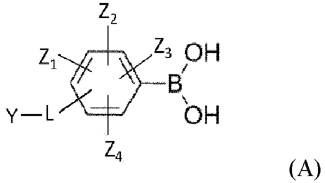 wherein:
Z₁, Z₂, Z₃ and Z₄ are each independently selected from a group consisting of H, an electron-withdrawing group or an electron-donating group, preferably each independently selected from a group consisting of hydrogen, C₁₋₆ alkyl, halogen, nitro, carboxyl and amino, more preferably each independently selected from a group consisting of hydrogen, C₁₋₆ alkyl and halogen;
L is absent, or a linker group connecting benzene ring and Y, preferably the linker group is selected from a group consisting of: C₁₋₆ alkylene, -(CH₂)ₙ-NR'-CO-aryl-N=N-aryl-NR'-(CH₂)ₒ-, -(CH₂)ₙ-NR'-COO-(CH₂)ₘ-aryl-(CH₂)ₒ-NR'-(CH₂)ₚ-, -(CH₂)ₙ-NR'-CO-, -(CH₂)ₙ-CO-NR'-, -(CH2).-NR'-aryl-(CH2)m-, -(CH2),-NR'-(CH2)m-aryl-(CH2)o-, -(CH₂)ₙ-NR'-(CH₂)ₘ-aryl-N=N-aryl-(CH₂)ₒ- and -C₂₋₆ alkenylene-aryl-(CH₂)ₒ-; wherein n, m, o and p are each independently an integer of 0-4; R' is H or C₁₋₄ alkyl; the aryl is selected from a group consisting of phenyl, naphthyl, anthracenyl and phenanthrenyl, and the aryl is optionally substituted by 1-3 substituents selected from a group consisting of halogen, C₁₋₄ alkyl and C₁₋₄ alkoxy; and
Y is selected from a group consisting of amino, hydroxyl, carboxyl, sulfonic acid, mercapto, alkenyl, alkynyl, azido, tetrazine, halogen, hydrazine, epoxy, isocyanate and isothiocyanate; preferably hydroxyl, amino, carboxyl or mercapto; and/or
(3) the size of the nanoparticle pore blocking agent matches the mesopore size of the mesoporous silica nanoparticle; preferably, the nanoparticle pore blocking agent is selected from a group consisting of metal oxide nanoparticles, metal sulfide nanoparticles, metal nanoparticles, quantum dots, block copolymers, natural polymers and biomacromolecules, preferably metal oxide nanoparticles such as zinc oxide nanoparticles.

3. The mesoporous silica nanomaterial for controlled release according to claim 1, wherein in the mesoporous silica nanoparticles functionalized by the polyhydroxy compound, an aminoalkyltrialkoxysilane is connected to the surface of the mesoporous silica nanoparticles, the polyhydroxy compound is connected to the aminoalkyltrialkoxysilane via an amide bond;
preferably, the mesoporous silica nanoparticle functionalized by the polyhydroxy compound has a structure shown in the following formula I: 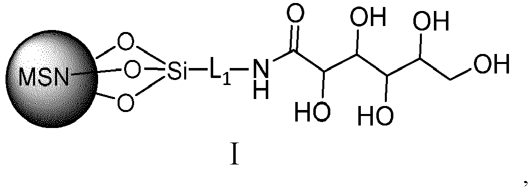 wherein MSN refers to the mesoporous silica nanoparticle; L₁ is a linker group; preferably, L₁ is a C₁₋₆ alkylene group.

4. The mesoporous silica nanomaterial for controlled release according to claim 1, wherein the nanoparticle pore blocking agent functionalized by phenylboronic acid has a structure shown in the following formula III or IV: 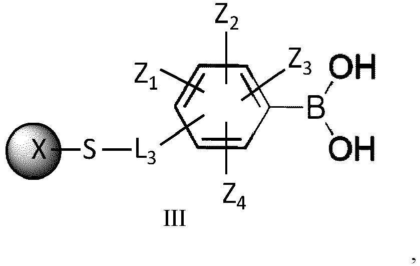 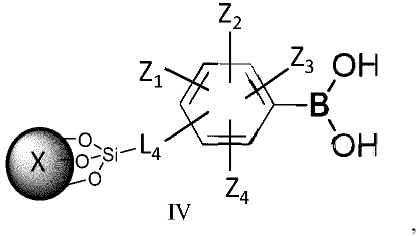 wherein X refers to the nanoparticle pore blocking agent; Z₁, Z₂, Z₃ and Z₄ are each independently selected from a group consisting of hydrogen, electron-withdrawing group and electron-donating group, preferably each independently selected from a group consisting of hydrogen, C₁₋₆ alkyl, halogen, nitro, carboxyl and amino, more preferably each independently selected from a group consisting of hydrogen, C₁₋₆ alkyl and halogen; L₃ and L₄ are each independently absent or a linker group, preferably, the linker group is selected from a group consisting of C₁₋₆ alkylene, -(CH₂)ₙ-NR'-CO-aryl-N=N-aryl-NR'-(CH₂)ₒ-, -(CH₂)ₙ-NR'-COO-(CH₂)ₘ-aryl-(CH₂)ₒ-NR'-(CH₂)ₚ-, -(CH₂)ₙ-NR'-CO-, -(CH₂)ₙ-CO-NR'-, -(CH₂)ₙ-NR'-aryl-(CH₂)ₘ-, -(CH₂)ₙ-NR'-(CH₂)ₘ-aryl-(CH₂)ₒ-, -(CH₂)ₙ-NR'-(CH₂)ₘ-aryl-N=N-aryl-(CH₂)ₒ-, and -C₂₋₆ alkenylene-aryl-(CH₂)ₒ-; wherein n, m, o and p are each independently an integer of 0-4; R' is H or C₁₋₄ alkyl; the aryl is selected from phenyl, naphthyl, anthracenyl and phenanthrenyl, and is optionally substituted by 1-3 substituents selected from a group consisting of halogen, C₁₋₄ alkyl and C₁₋₄ alkoxy;
preferably, the nanoparticle pore blocking agent functionalized by phenylboronic acid is: 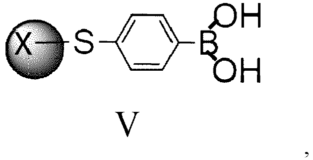 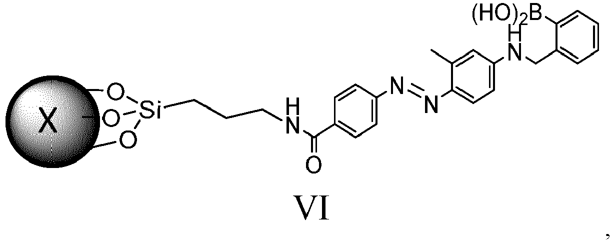 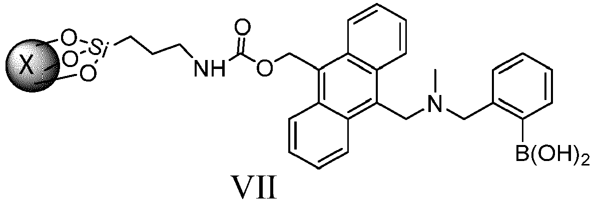 or 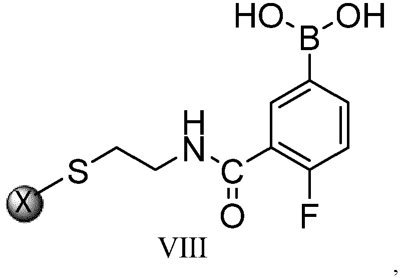 wherein X refers to the nanoparticle pore blocking agent.

5. A method for preparing the mesoporous silica nanomaterial for controlled release according to any one of claims 1-4, wherein the method comprises the following steps:
(1) providing mesoporous silica nanoparticles functionalized by a polyhydroxy compound and a pore blocking agent functionalized by phenylboronic acid; and
(2) dispersing the mesoporous silica nanoparticles functionalized by the polyhydroxy compound and the pore blocking agent functionalized by phenylboronic acid in a solvent, and reacting to obtain the mesoporous silica nanomaterial for controlled release.

6. A functionally active molecule nano-drug delivery system, wherein the functionally active molecule nano-drug delivery system comprises the mesoporous silica nanomaterial for controlled release according to any one of claims 1-4 and a functionally active molecule; preferably, the size of the functionally active molecule is smaller than the mesopore size of the mesoporous silica nanoparticle; preferably, the functionally active molecule is loaded inside the mesoporous silica nanoparticle; preferably, the functionally active molecule comprises a diabetes therapeutic agent; preferably, the diabetes therapeutic agent is various types of insulin or its biologically active analogues; preferably, the insulin or its biologically active analogue is selected from a group consisting of human insulin, recombinant human insulin, insulin from non-human animals, rapid-acting insulin, rapid-acting insulin analogs, intermediate-acting insulin and long-acting insulin.

7. A method for preparing the functionally active molecule nano-drug delivery system according to claim 6, wherein the method comprises the following steps:
(1) providing mesoporous silica nanoparticles functionalized by a polyhydroxy compound and a pore blocking agent functionalized by phenylboronic acid;
(2) dispersing the mesoporous silica nanoparticles functionalized by the polyhydroxy compound in a solvent containing a functionally active molecule to allow the mesoporous silica nanoparticles to be loaded with the functionally active molecules; and
(3) adding the pore blocking agent functionalized by phenylboronic acid to the reaction solution in the step (2) for reaction to obtain the functionally active molecule nano-drug delivery system.

8. A kit, wherein the kit comprises mesoporous silica nanoparticles functionalized by a polyhydroxy compound and a nanoparticle pore blocking agent functionalized by phenylboronic acid; preferably, the mesoporous silica nanoparticle functionalized by the polyhydroxy compound is as defined in claim 2 or 3; preferably, the nanoparticle pore blocking agent functionalized by phenylboronic acid is as defined in claim 2 or 4; optionally, the kit further comprises the functionally active molecule according to claim 6; preferably, the mesoporous silica nanoparticle functionalized by the polyhydroxy compound, the nanoparticle pore blocking agent functionalized by phenylboronic acid, and the optional functionally active molecule contained in the kit are respectively stored in different containers.

9. A microneedle or a microneedle array patch, wherein the microneedle or the microneedle array patch comprises the mesoporous silica nanomaterial for controlled release according to any one of claims 1-4 or the functionally active molecule nano-drug delivery system according to claim 6.

10. Use of the mesoporous silica nanomaterial for controlled release according to any one of claims 1-4 in the manufacture of a medicine or a kit for treating diabetes or controlling blood sugar level in a patient; or
use of the functionally active molecule nano-drug delivery system according to claim 6 in the manufacture of a medicine or a kit for treating diabetes or controlling blood sugar levels in a patient, wherein the functionally active molecule comprises a diabetes therapeutic agent; or
use of the mesoporous silica nanomaterial for controlled release according to any one of claims 1-4 or the functionally active molecule nano-drug delivery system according to claim 6 in the manufacture of a medicine or a diagnostic reagent or a diagnostic kit for controlled release of a drug.
